# EUROPÄISCHE PATENTSCHRIFT

(11) **EP 3 300 675 B1**
(45) Veröffentlichungstag und Bekanntmachung des Hinweises auf die Patenterteilung: **07.08.2024**
(21) Anmeldenummer: 17192042.4
(22) Anmeldetag: 20.09.2017
(51) Int. Cl.: A61B 17/29

(54) **KOMPONENTE FÜR EIN MEDIZINISCHES INSTRUMENT UND MEDIZINISCHES INSTRUMENT**
COMPONENTS FOR A MEDICAL INSTRUMENT AND MEDICAL INSTRUMENT
COMPOSANTS POUR UN INSTRUMENT MÉDICAL ET INSTRUMENT MÉDICAL

(30) Priorität: 28.09.2016 DE 102016118304
(43) Veröffentlichungstag der Anmeldung: 04.04.2018
(73) Patentinhaber: Karl Storz SE & Co. KG, 78532 Tuttlingen (DE)
(72) Erfinder: STEFAN, Jochen, 78532 Tuttlingen (DE); KÄRCHER, Daniel, 78532 Tuttlingen (DE)

(56) Entgegenhaltungen:
- EP-A1- 2 269 522
- EP-A1- 2 561 816
- DE-A1- 19 731 453
- DE-A1- 4 311 770
- US-A- 4 569 131

## Beschreibung

Die vorliegende Erfindung ist auf eine Komponente für ein medizinisches Instrument und auf ein medizinisches Instrument bezogen. Die vorliegende Erfindung ist insbesondere auf Maßnahmen bezogen, die unter bestimmten Umständen eine Zerstörung eines medizinischen Instruments durch unsachgemäße Verwendung oder Handhabung verhindern können.

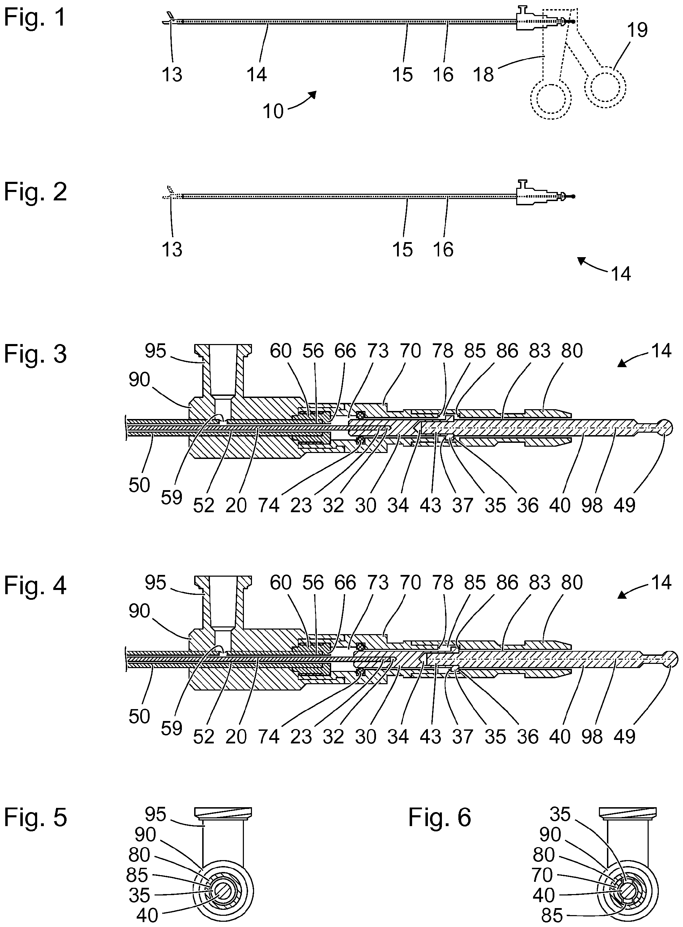

In DE 197 31 453 A1 (ähnlich oder gleich: WO 99/04703 A1 und EP 0 925 037 B1) ist ein chirurgisches Instrument 10 mit einer Vorrichtung 30 zur Begrenzung der Kraftübertragung beschrieben (Spalte 7, Zeilen 39 bis 41). Diese Vorrichtung 30 verbindet einen ersten Abschnitt 32 und einen zweiten Abschnitt 54 eines im Übrigen stabförmigen Betätigungselements 26 (Figuren 1, 2). Die Vorrichtung 30 umfasst ein näherungsweise hohlzylinderförmiges Gehäuse 38, das mit dem ersten Abschnitt 32 des stabförmigen Betätigungselements 26 starr verbunden ist und sich axial erstreckende Nuten (Spalte 7, Zeilen 45 bis 55) und einen Innenkonus (Spalte 8, Zeilen 14 bis 16; Figur 4) aufweist. Die Vorrichtung 30 umfasst ferner einen Konus 56, der mit dem zweiten Abschnitt 54 des stabförmigen Betätigungselements 26 verbunden ist, eine konische Keilfläche 57 aufweist und in dem hohlzylinderförmigen Gehäuse 38 so angeordnet ist, dass der Konus 56 an dem Innenkonus 64 anliegt (Spalte 8, Zeilen 7 bis 23). Bei Überschreiten einer vorbestimmten Kraft wird das hohlzylinderförmige Gehäuse 38 verformt und der Konus 56 aus diesem teilweise heraus gezogen (Spalte 8, Zeilen 33 bis 53).

In DE 10 2009 031 262 A1 und EP 2 269 522 A1 ist ein medizinisches Instrument 1 beschrieben, bei dem in der Handhabe 3 eine Kraftbegrenzungsvorrichtung 8 vorgesehen ist, die zwei miteinander gekoppelte Komponenten bei Überschreiten einer Grenzlast reversibel trennt (Absatz [0024] von EP 2 269 522 A1).

In DE 10 2011 007 121 A1, EP 2 510 888 A1, DE 10 2011 007 119 A1 und

EP 2 510 889 A1 ist eine Handhabungseinrichtung für ein mikroinvasiv-chirurgisches Instrument beschrieben. Eine vorgespannte Druckfeder 564 in einem becherförmigen Bauteil 562 begrenzt eine von einem Stab 561 auf eine Übertragungsstange 40 zu übertragende Zugkraft (Absatz [0083] von EP 2 510 888 A1; Figur 3).

In DE 10 2012 002 770 A1 und EP 2 626 018 A2 ist ein medizinisches Instrument beschrieben, bei dem in der Handhabe 3 eine Kraftbegrenzungsvorrichtung 8 vorgesehen ist, die bei Überschreiten einer Grenzlast die übertragene Kraft reversibel begrenzt (Absatz [0026] von EP 2 626 018 A2.)

In DE 197 31 453 A1 ist ein chirurgisches Instrument 10 mit einer Vorrichtung 30 zur Begrenzung der Kraftübertragung beschrieben (Spalte 7, Zeilen 39 bis 41, Figuren). Diese Vorrichtung 30 verbindet einen ersten, distalen Abschnitt 32 und einen zweiten, proximalen Abschnitt 54 eines im Übrigen stabförmigen Betätigungselements 26 (Spalte 7, Zeilen 45 bis 47; Spalte 8, Zeilen 7 bis 8; Figuren 1, 2). Die Vorrichtung 30 umfasst ein als näherungsweise hohlzylinderförmig bezeichnetes Gehäuse 38, das mit dem ersten Abschnitt 32 des stabförmigen Betätigungselements 26 starr verbunden ist und axial sich erstreckende Nuten (Spalte 7, Zeilen 45 bis 55) sowie einen Innenkonus 64 (Spalte 8, Zeilen 14 bis 16; Figur 4) aufweist. Die Vorrichtung 30 umfasst ferner einen Konus 56, der mit dem zweiten Abschnitt 54 des stabförmigen Betätigungselements 26 verbunden ist, eine konische Keilfläche 57 aufweist und in dem Gehäuse 38 so angeordnet ist, dass der Konus 56 an dem Innenkonus 64 anliegt (Spalte 8, Zeilen 7 bis 23). Bei Überschreiten einer vorbestimmten Kraft wird das Gehäuse 38 verformt und der Konus 56 aus diesem teilweise heraus gezogen (Spalte 8, Zeilen 33 bis 53).

In EP 2 561 816 A1 ist ein Werkzeug für ein mikroinvasiv-chirurgisches Instrument beschrieben.

In EP 2 269 522 A1 ist ein medizinisches Instrument 1 beschrieben, bei dem in der Handhabe 3 eine Kraftbegrenzungsvorrichtung 8 vorgesehen ist, die zwei miteinander gekoppelte Komponenten bei Überschreiten einer Grenzlast reversibel trennt (Absatz [0024]).

In DE 43 11 770 A1 ist eine Greifpinzette 1 mit einem in einen Körperhohlraum eines Patienten einführbaren Einführbereich 2 beschrieben (Spalte 4, Zeilen 14 bis 22, Figur 1). In einer Umhüllung 24 ist ein Betätigungsschaft 10 angeordnet, der Greifteile 27a, 27b in einem Pinzettenbereich 3 mit einem Bedienbereich 4 verbindet (Spalte 4, Zeilen 31 bis 50 und Zeilen 66 bis 68, Figuren 3, 4). Das proximale Ende des Übertragungsschafts 10 wird durch einen Kopplungsstab 41 gebildet (Spalte 5, Zeilen 46 bis 56, Figur 4). Eine Stufe zwischen einem Bereich 41a des Kopplungsstabs 41 mit großem Durchmesser und einem Bereich 41b des Kopplungsstabs 41mit kleinem Durchmesser und eine Stufe zwischen einer Stabhaltebohrung 37 und einer Durchgangsbohrung 38 in dem Bedienbereich 4 bilden eine Stopper-Vorrichtung 12, die die Bewegung des Betätigungsschafts 10 nach proximal beschränkt (Spalte 6, Zeilen 19 bis 28, Figur 4).

In US 4,569,131 A1 ist ein Werkzeug mit einer Handhabungseinrichtung, einem austauschbaren Schaft und einem Paar schwenkbar verbundener Bauteile beschrieben. Um ein Überschließen, also ein Überstehen der Schneidkante eines schwenkbaren Bauteils gegen über dem anderen schwenkbaren Bauteil zu vermeiden, ist ein ringförmiger Begrenzungsanschlag vorgesehen, der zwischen zwei Anschlägen bewegbar ist.

Eine Aufgabe der vorliegenden Erfindung besteht darin, eine verbesserte Komponente für ein medizinisches Instrument, ein verbessertes medizinisches Instrument und ein verbessertes Verfahren zum Herstellen einer Komponente für ein medizinisches Instrument zu schaffen.

Diese Aufgabe wird durch die Gegenstände der unabhängigen Ansprüche gelöst.

Weiterbildungen sind in den abhängigen Ansprüchen angegeben.

Eine Komponente für ein medizinisches Instrument umfasst einen Schaft, eine Übertragungseinrichtung, die in dem Schaft bewegbar ist, zum Übertragen einer Kraft von einer mit dem proximalen Ende der Komponente gekoppelten Handhabungseinrichtung zu einem Werkzeug an dem distalen Ende der Komponente, eine Anschlagfläche an dem Schaft und eine Anschlagfläche an der Übertragungseinrichtung, wobei die Anschlagfläche des Schafts und die Anschlagfläche der Übertragungseinrichtung so angeordnet sind, dass ein mechanischer Kontakt zwischen der Anschlagfläche und der Übertragungseinrichtung und der Anschlagfläche des Schafts eine Bewegung der Übertragungseinrichtung relativ zu dem Schaft nach proximal begrenzt.

Die Komponente ist vorgesehen und ausgebildet, um mit einer oder mehreren weiteren Komponenten so mechanisch verbunden und / oder gekoppelt zu werden, dass ein medizinisches Instrument gebildet wird. Die Komponente ist insbesondere eine Komponente für ein mikroinvasives medizinisches Instrument, wobei der Schaft einen Durchmesser von wenigen Millimetern oder weniger und eine Länge von einigen Zentimetern oder wenigen oder einigen Dezimetern aufweist. Der Schaft kann beispielsweise dafür vorgesehen und ausgebildet sein, um in einen Arbeitskanal eines Endoskops eingeführt zu werden.

Das proximale Ende des Schafts ist insbesondere zur zerstörungsfrei lösbaren oder zur dauerhaften bzw. nicht zerstörungsfrei lösbaren mechanischen Verbindung mit einer Handhabungseinrichtung vorgesehen und ausgebildet. Das proximale Ende der Übertragungseinrichtung ist insbesondere zur zerstörungsfrei lösbaren oder dauerhaften bzw. nicht zerstörungsfrei lösbaren mechanischen Kopplung mit einem manuell bewegbaren Teil einer Handhabungseinrichtung vorgesehen und ausgebildet.

Die Komponente kann an ihrem distalen Ende ein Werkzeug aufweisen, das mit dem distalen Ende des Schafts insbesondere starr verbunden ist, und das einen bewegbaren Teil (beispielsweise eine schwenkbare Branche oder ein schwenkbares Maulteil) aufweist. Dabei ist der bewegbare Teil des Werkzeugs derart unmittelbar oder mittelbar mit dem distalen Ende der Übertragungseinrichtung mechanisch gekoppelt, dass eine Bewegung der Übertragungseinrichtung relativ zu dem Schaft mit einer Bewegung des bewegbaren Teils des Werkzeugs relativ zu einem anderen Teil des Werkzeugs einhergeht. Das Werkzeug kann mit dem distalen Ende des Schafts und mit dem distalen Ende der Übertragungseinrichtung zerstörungsfrei lösbar oder dauerhaft bzw. nicht zerstörungsfrei lösbar mechanisch verbunden und gekoppelt sein.

Alternativ kann die Komponente kein Werkzeug aufweisen, jedoch zur zerstörungsfrei lösbaren oder zur dauerhaften bzw. nicht zerstörungsfrei lösbaren mechanischen Verbindung und Kopplung eines Werkzeugs mit dem distalen Ende des Schafts und mit dem distalen Ende der Übertragungseinrichtung vorgesehen und ausgebildet sein.

Die Übertragungseinrichtung ist insbesondere in Richtung parallel zur Längsachse des Schafts relativ zu dem Schaft bewegbar. Die Übertragungseinrichtung ist insbesondere zum Übertragen einer Zugkraft von einem mit dem proximalen Ende der Übertragungseinrichtung gekoppelten bewegbaren Teil einer Handhabungseinrichtung zu einem mit dem distalen Ende der Übertragungseinrichtung gekoppelten bewegbaren Teil eines Werkzeugs vorgesehen und ausgebildet. Alternativ oder zusätzlich kann die Übertragungseinrichtung zum Übertragen einer Schubkraft von einem mit dem proximalen Ende der Übertragungseinrichtung gekoppelten bewegbaren Teil einer Handhabungseinrichtung zu einem mit dem distalen Ende der Übertragungseinrichtung gekoppelten bewegbaren Teil eines Werkzeugs vorgesehen und ausgebildet sein.

Das distale Ende der Übertragungseinrichtung ist insbesondere mit einem bewegbaren Teil eines Werkzeugs derart koppelbar oder derart gekoppelt, dass eine Bewegung der Übertragungseinrichtung nach proximal mit der vorgesehenen Wirkbewegung des bewegbaren Teils des Werkzeugs einhergeht. In diesem Fall wird die für die Wirkbewegung des bewegbaren Teils des Werkzeugs erforderliche Kraft durch eine Zugkraft bzw. Zugspannung in der Übertragungseinrichtung übertragen. Die Wirkbewegung des bewegbaren Teils eines Werkzeugs ist die Bewegung, bei der das Werkzeug die vorgesehene Wirkung entfaltet. Bei einer Zange oder einem anderen Werkzeug zum Greifen, Halten, Quetschen, Stanzen oder Schneiden ist dies in der Regel die schließende Bewegung, bei der zwei (in manchen Fällen mehr) Maulteile bzw. Branchen aufeinander zu bewegt werden. In anderen Fällen kann die Wirkbewegung eine Bewegung sein, bei der Maulteile bzw. Branchen voneinander wegbewegt werden, beispielsweise bei einem Werkzeug zum Spreizen oder Weiten eines Gefäßes oder eines anderen Hohlraums.

Alternativ zu einer Anordnung der Anschlagfläche des Schafts und der Anschlagfläche der Übertragungseinrichtung derart, dass ein mechanischer Kontakt zwischen der Anschlagfläche der Übertragungseinrichtung und der Anschlagfläche des Schafts eine Bewegung der Übertragungseinrichtung relativ zu dem Schaft nach proximal begrenzt, können die Anschlagfläche des Schafts und die Anschlagfläche der Übertragungseinrichtung derart angeordnet sein, dass ein mechanischer Kontakt zwischen der Anschlagfläche der Übertragungseinrichtung und der Anschlagfläche des Schafts eine Bewegung der Übertragungseinrichtung relativ zu dem Schaft nach distal begrenzt.

Bei einer Komponente, wie sie hier beschrieben ist, sind insbesondere die Anschlagfläche des Schafts nach distal und die Anschlagfläche der Übertragungseinrichtung nach proximal orientiert.

Bei einer Komponente, wie sie hier beschrieben ist, sind insbesondere die Bewegung des distalen Endes der Übertragungseinrichtung relativ zu dem distalen Ende des Schafts nach proximal so begrenzt und die Anschlagfläche des Schafts und die Anschlagfläche der Übertragungseinrichtung so angeordnet, dass ein mechanischer Kontakt zwischen der Anschlagfläche der Übertragungseinrichtung und der Anschlagfläche des Schafts eine elastische Verformung der Übertragungseinrichtung durch eine von der Übertragungseinrichtung übertragene Kraft auf einen vorbestimmten Maximalwert begrenzt, wobei der vorbestimmte Maximalwert der elastischen Verformung und elastische Eigenschaften der Übertragungseinrichtung so gewählt sind, dass bei der Kraft, die bei dem vorbestimmten Maximalwert der elastischen Verformung der Übertragungseinrichtung vorliegt, eine Zerstörung oder Schädigung des Schafts, der Übertragungseinrichtung und eines mit dem distalen Ende des Schafts verbundenen und mit dem distalen Ende der Übertragungseinrichtung gekoppelten Werkzeugs ausgeschlossen ist.

Die Bewegung des distalen Endes der Übertragungseinrichtung relativ zu dem distalen Ende des Schafts ist insbesondere durch ein Werkzeug, das mit dem distalen Ende des Schafts mechanisch verbunden und mit dem distalen Ende der Übertragungsrichtung mechanisch gekoppelt ist, begrenzt. Wenn die Komponente kein Werkzeug am distalen Ende umfasst, sondern zur mechanischen Verbindung und Kopplung mit einem Werkzeug vorgesehen und ausgebildet ist, ist die Bewegung des distalen Endes der Übertragungseinrichtung relativ zu dem distalen Ende des Schafts insbesondere nur dann nach proximal begrenzt oder nur dann in der Weise, die für die hier beschriebene Begrenzung der übertragenen Kraft auf den vorbestimmten Maximalwert erforderlich ist, begrenzt, wenn das distale Ende des Schafts mit dem für die Komponente vorgesehenen Werkzeug oder mit einem von mehreren für die Komponente vorgesehenen Werkzeugen in der vorgesehenen Weise verbunden und ein oder mehrere bewegbare Teile des Werkzeugs in der vorgesehenen Weise mit dem distalen Ende der Übertragungseinrichtung mechanisch gekoppelt sind.

Bei einer Komponente, wie sie hier beschrieben ist, sind die Anschlagfläche des Schafts und die Anschlagfläche der Übertragungseinrichtung insbesondere so angeordnet, dass ein mechanischer Kontakt zwischen der Anschlagfläche der Übertragungseinrichtung und der Anschlagfläche des Schafts bei der vorgesehenen Verwendung eine elastische Verformung der Übertragungseinrichtung durch eine von der Übertragungseinrichtung übertragene Kraft auf einen vorbestimmten Maximalwert begrenzt, wobei der vorbestimmte Maximalwert der elastischen Verformung und elastische Eigenschaften der Übertragungseinrichtung so gewählt sind, dass bei der Kraft, die bei dem vorbestimmten Maximalwert der elastischen Verformung der Übertragungseinrichtung vorliegt, eine Zerstörung oder Schädigung des Schafts, der Übertragungseinrichtung und eines mit dem distalen Ende des Schafts verbundenen und mit dem distalen Ende der Übertragungseinrichtung gekoppelten Werkzeugs ausgeschlossen ist.

Auch eine plastische Verformung der Übertragungseinrichtung, des Schafts oder eines mit dem distalen Ende des Schafts und dem distalen Ende der Übertragungseinrichtung gekoppelten Werkzeugs ist eine Schädigung der Übertragungseinrichtung, des Schafts bzw. des Werkzeugs. Ein Ausschluss einer Zerstörung oder Schädigung des Schafts, der Übertragungseinrichtung und eines mit dem distalen Ende des Schafts und dem distalen Ende der Übertragungseinrichtung gekoppelten Werkzeugs bedeutet somit auch, dass die Übertragungseinrichtung, der Schaft und das Werkzeug jeweils lediglich elastisch und nicht plastisch verformt werden.

Bei einer Komponente, wie sie hier beschrieben ist, ist eine Zerstörung oder Schädigung des Schafts, der Übertragungseinrichtung und eines mit dem distalen Ende des Schafts gekoppelten Werkzeugs insbesondere auch dann ausgeschlossen, wenn das Werkzeug in einem ganz geöffneten oder in einem ganz geschlossenen oder in einem beliebigen Zustand blockiert ist.

Ein mit dem distalen Ende des Schafts verbundenes und mit dem distalen Ende der Übertragungseinrichtung gekoppeltes Werkzeug zum Schneiden, Greifen, Halten, Quetschen oder Stanzen kann beispielsweise durch einen harten oder bei der erzeugbaren Kraft nicht nachgebenden Gegenstand in seiner ganz geöffneten Konfiguration blockiert werden. Wenn die Übertragungseinrichtung mit dem bewegbaren Teil des Werkzeugs derart gekoppelt ist, dass zum Schließen des Werkzeugs eine durch die Übertragungseinrichtung zu übertragende Zugkraft erforderlich ist, ist die Übertragungseinrichtung dann maximal verformt, nämlich gedehnt, wenn das Werkzeug im ganz geöffneten Zustand blockiert ist und die Anschlagfläche der Übertragungseinrichtung an der Anschlagfläche des Schafts anliegt. Die Anschlagfläche der Übertragungseinrichtung und die Anschlagfläche des Schafts sind so positioniert und die elastischen Eigenschaften der Übertragungseinrichtung sind so gewählt, dass auch in diesem Fall der Schaft, die Übertragungseinrichtung und ein mit dem distalen Ende des Schafts gekoppeltes Werkzeug nicht geschädigt oder zerstört werden.

Bei einer Komponente, wie sie hier beschrieben ist, sind insbesondere die Anschlagfläche des Schafts nahe dem proximalen Ende des Schafts und die Anschlagfläche der Übertragungseinrichtung nahe dem proximalen Ende der Übertragungseinrichtung angeordnet.

Der Abstand der Anschlagfläche des Schafts von dem proximalen Ende des Schafts beträgt insbesondere nicht mehr als ein Fünftel oder nicht mehr als ein Zehntel der Länge des Schafts. Der Abstand der Anschlagfläche der Übertragungseinrichtung von dem proximalen Ende der Übertragungseinrichtung beträgt insbesondere nicht mehr als ein Fünftel oder nicht mehr als ein Zehntel der Länge der Übertragungseinrichtung.

Bei einer Komponente, wie sie hier beschrieben ist, sind die Anschlagfläche des Schafts und die Anschlagfläche der Übertragungseinrichtung insbesondere in einem proximalen Bereich des Schafts, in dem der Schaft einen vergrößerten Durchmesser oder einen vergrößerten Querschnitt aufweist, angeordnet.

Bei einer Komponente, wie sie hier beschrieben ist, sind die Anschlagfläche des Schafts und die Anschlagfläche der Übertragungseinrichtung insbesondere in einem Bereich des Schafts, der zur Aufnahme in einer Handhabungseinrichtung vorgesehen ist, angeordnet.

Der Schaft weist insbesondere einen langen und dünnen rohrförmigen Abschnitt und einen im Vergleich zu dem rohrförmigen Abschnitt wesentlich dickeren proximalen Abschnitt auf. Der lange und dünne rohrförmige Abschnitt des Schafts erstreckt sich in der Regel über mindestens zwei Drittel oder mindestens vier Fünftel oder mindestens neun Zehntel der Länge des Schafts und hat einen Durchmesser von wenigen Millimetern oder weniger. Durchmesser und Querschnitt des proximalen Abschnitts des Schafts sind in der Regel deutlich größer als Durchmesser und Querschnitt des langen und dünnen rohrförmigen Abschnitts. In dem proximalen Abschnitt steht mehr Bauraum für die Anschlagfläche des Schafts und für die Anschlagfläche der Übertragungseinrichtung zur Verfügung.

Bei einer Komponente, wie sie hier beschrieben ist, weist die Übertragungseinrichtung insbesondere in einem Abschnitt distal der Anschlagfläche der Übertragungseinrichtung ihre minimale Querschnittsfläche auf, wobei alle Querschnittsflächen der Übertragungseinrichtung proximal der Anschlagfläche der Übertragungseinrichtung größer sind als die minimale Querschnittsfläche der Übertragungseinrichtung.

Die Querschnittsfläche der Übertragungseinrichtung ist insbesondere proximal der Anschlagfläche der Übertragungseinrichtung durchgehend wesentlich größer als distal der Anschlagfläche der Übertragungseinrichtung. Die Querschnittsfläche der Übertragungseinrichtung ist beispielsweise proximal der Anschlagfläche der Übertragungseinrichtung durchgehend mindestens um einen Faktor 5 oder mindestens um einen Faktor 10 oder mindestens um einen Faktor 20 größer als distal der Anschlagfläche der Übertragungseinrichtung. Die Übertragungseinrichtung weist insbesondere distal der Anschlagfläche der Übertragungseinrichtung weitgehend einen konstanten kreisförmigen Querschnitt mit einem Durchmesser von weniger als 1 mm auf. Proximal der Anschlagfläche der Übertragungseinrichtung weist die Übertragungseinrichtung insbesondere durchgehend einen kreisförmigen oder anderen Querschnitt mit einer Querschnittsfläche von mehreren Quadratmillimetern auf.

Eine Ausgestaltung der Übertragungseinrichtung derart, dass ihre Querschnittsfläche proximal der Anschlagfläche der Übertragungseinrichtung deutlich größer ist als distal, ermöglicht eine große Robustheit der Übertragungseinrichtung proximal der Anschlagfläche der Übertragungseinrichtung und deshalb ein geringes Risiko einer Zerstörung oder Schädigung durch eine zu große Kraft.

Bei einer Komponente, wie sie hier beschrieben ist, weist die Übertragungseinrichtung insbesondere in einem Abschnitt distal der Anschlagfläche der Übertragungseinrichtung ihre minimale Zugfestigkeit auf, wobei die Zugfestigkeit der Übertragungseinrichtung proximal der Anschlagfläche der Übertragungseinrichtung durchgehend größer ist als die minimale Zugfestigkeit der Übertragungseinrichtung.

Die Zugfestigkeit der Übertragungseinrichtung ist proximal der Anschlagfläche der Übertragungseinrichtung insbesondere wesentlich größer als die minimale Zugfestigkeit der Übertragungseinrichtung. Die Zugfestigkeit der Übertragungseinrichtung ist proximal der Anschlagfläche der Übertragungseinrichtung beispielsweise mindestens um einen Faktor 5 oder um einen Faktor 10 oder um einen Faktor 20 größer als die minimale Zugfestigkeit der Übertragungseinrichtung.

Bei einer Komponente, wie sie hier beschrieben ist, ist die Übertragungseinrichtung insbesondere distal der Anschlagfläche der Übertragungseinrichtung bis zu einer ersten Maximalkraft elastisch verformbar, wobei die Übertragungseinrichtung proximal der Anschlagfläche der Übertragungseinrichtung bis zu einer zweiten Maximalkraft elastisch verformbar ist, und wobei die zweite Maximalkraft größer ist als die erste Maximalkraft.

Die Maximalkraft, bis zu der die Übertragungseinrichtung an einem bestimmten Punkt elastisch verformbar ist, ist die größte Kraft, bei der die Übertragungseinrichtung an dem bestimmten Punkt noch nicht plastisch verformt wird. Die zweite Maximalkraft ist insbesondere deutlich größer als die erste Maximalkraft. Die zweite Maximalkraft ist insbesondere mindestens um einen Faktor 2 oder mindestens um einen Faktor 5 oder mindestens um einen Faktor 10 oder mindestens um einen Faktor 20 oder mindestens um einen Faktor 50 größer als die erste Maximalkraft.

Durch Ausgestaltung und Anordnung der Anschlagfläche der Übertragungseinrichtung und der Anschlagfläche des Schafts und Wahl elastischer Eigenschaften der Übertragungseinrichtung kann eine Zerstörung oder Schädigung der Übertragungseinrichtung sowie des Schafts und eines Werkzeugs am distalen Ende der Komponente verhindert werden. Eine proximal der Anschlagfläche der Übertragungseinrichtung wesentlich größere mechanische Robustheit der Übertragungseinrichtung kann auch das Risiko einer Zerstörung oder Schädigung der Übertragungseinrichtung proximal der Anschlagfläche der Übertragungseinrichtung deutlich reduzieren.

Bei einer Komponente, wie sie hier beschrieben ist, kann die Übertragungseinrichtung insbesondere weder nach proximal noch nach distal aus dem Schaft entnommen werden ohne den Schaft zu zerlegen.

Eine Komponente, wie sie hier beschrieben ist, umfasst insbesondere ferner eine weitere Anschlagfläche an dem Schaft und eine weitere Anschlagfläche an der Übertragungseinrichtung, wobei die weitere Anschlagfläche des Schafts und die weitere Anschlagfläche der Übertragungseinrichtung so angeordnet sind, dass ein mechanischer Kontakt zwischen der weiteren Anschlagfläche der Übertragungseinrichtung und der weiteren Anschlagfläche des Schafts eine Bewegung der Übertragungseinrichtung relativ zu dem Schaft nach distal begrenzt.

Die weitere Anschlagfläche des Schafts ist insbesondere nahe dem proximalen Ende des Schafts und insbesondere nahe der Anschlagfläche des Schafts angeordnet. Die weitere Anschlagfläche der Übertragungseinrichtung ist insbesondere nahe dem proximalen Ende der Übertragungseinrichtung und insbesondere nahe der Anschlagfläche der Übertragungseinrichtung angeordnet. Insbesondere sind die weitere Anschlagfläche des Schafts nach proximal und die weitere Anschlagfläche der Übertragungseinrichtung nach distal orientiert.

Die Begrenzung der Bewegung der Übertragungseinrichtung relativ zu dem Schaft nach distal kann eine Überlastung, Schädigung oder Zerstörung der Übertragungseinrichtung, des Schafts oder eines Werkzeugs am distalen Ende der Komponente durch eine zu hohe durch die Übertragungseinrichtung übertragene Schubkraft verhindern. Insbesondere kann eine Stauchung der Übertragungseinrichtung distal der weiteren Anschlagfläche der Übertragungseinrichtung verhindert werden.

Bei einer Komponente, wie sie hier beschrieben ist, weist die Übertragungseinrichtung insbesondere einen nach außen ragenden Kragen auf, wobei der Schaft eine Nut, die einen Kanal, in dem die Übertragungseinrichtung angeordnet ist, erweitert, aufweist, wobei der Kragen der Übertragungseinrichtung in der Nut des Schafts angeordnet ist, wobei die Anschlagfläche des Schafts Teil der inneren Oberfläche der Nut ist, und wobei die Anschlagfläche der Übertragungseinrichtung Teil der Oberfläche des Kragens ist.

Eine nach proximal orientierte Oberfläche des Kragens kann die Anschlagfläche der Übertragungseinrichtung, eine nach distal orientierte Oberfläche der Nut kann die Anschlagfläche des Schafts bilden. Eine nach distal orientierte Oberfläche an dem Kragen kann die weitere Anschlagfläche der Übertragungseinrichtung, eine nach proximal orientierte Fläche in der Nut kann die weitere Anschlagfläche des Schafts bilden, um eine Bewegung der Übertragungseinrichtung relativ zu dem Schaft nach distal zu begrenzen.

Der Kragen ist insbesondere ringförmig oder kreisringförmig. In diesem Fall bildet insbesondere ein ringförmiger oder kreisringförmiger Bereich der Oberfläche des Kragens die Anschlagfläche der Übertragungseinrichtung.

Die Nut ist insbesondere ringförmig oder kreisringförmig und erweitert den Kanal, in dem die Übertragungseinrichtung angeordnet ist, ringförmig. In diesem Fall bildet insbesondere ein ringförmiger oder kreisringförmiger Bereich der inneren Oberfläche der Nut die Anschlagfläche des Schafts.

Bei einer Komponente, wie sie hier beschrieben ist, weist die Übertragungseinrichtung insbesondere einen Vorsprung, eine Nase, einen Steg, einen Zapfen oder einen anderen konvexen Bereich auf, wobei der Schaft eine Nut oder eine andere nischenförmige Ausnehmung, die von einem Kanal, in dem die Übertragungseinrichtung angeordnet ist, ausgeht, aufweist, wobei der Vorsprung oder die Nase oder der Steg oder der Zapfen oder der andere konvexe Bereich der Übertragungseinrichtung in der Nut oder der anderen nischenförmigen Ausnehmung des Schafts angeordnet ist, wobei die Anschlagfläche des Schafts Teil der inneren Oberfläche der Nut oder der anderen nischenförmigen Ausnehmung des Schafts ist, und wobei die Anschlagfläche der Übertragungseinrichtung Teil der Oberfläche des Vorsprungs oder der Nase oder des Stegs oder des Zapfens oder des anderen konvexen Bereichs ist.

Ferner können ein weiterer, nach proximal orientierter Teil der inneren Oberfläche der Nut oder der anderen nischenförmigen Ausnehmung des Schafts eine weitere Anschlagfläche des Schafts und ein weiterer, nach distal orientierter Teil der Oberfläche des Vorsprungs oder der Nase oder des Stegs oder des Zapfens oder des anderen konvexen Bereichs eine weitere Anschlagfläche der Übertragungseinrichtung bilden, um eine Bewegung der Übertragungseinrichtung relativ zu dem Schaft nach distal zu begrenzen.

Eine Komponente, wie sie hier beschrieben ist, umfasst insbesondere ferner ein Werkzeug an dem distalen Ende des Schafts, wobei das Werkzeug ein bewegbares Bauteil, das mit dem distalen Ende der Übertragungseinrichtung gekoppelt ist, aufweist.

Das Werkzeug kann mit dem distalen Ende des Schafts zerstörungsfrei lösbar oder dauerhaft bzw. nicht zerstörungsfrei lösbar verbunden sein. Das Werkzeug ist insbesondere zum Greifen, Fassen, Halten, Quetschen, Schneiden oder Stanzen ausgebildet. Das bewegbare Bauteil ist insbesondere eine Branche oder ein Maulteil des Werkzeugs. Das bewegbare Bauteil ist insbesondere derart mit der Übertragungseinrichtung gekoppelt, dass eine lineare Translationsbewegung der Übertragungseinrichtung in Richtung parallel zu ihrer Längsachse mit einer Schwenkbewegung des bewegbaren Bauteils einhergeht.

Ein medizinisches Instrument umfasst eine Komponente, wie sie hier beschrieben ist, und eine Handhabungseinrichtung, die mit dem proximalen Ende des Schafts gekoppelt oder koppelbar ist.

Ein Verfahren zum Herstellen einer Komponente für ein medizinisches Instrument umfasst einen Schritt des Hindurchführens eines proximalen Endes eines ersten Bauteils einer Übertragungseinrichtung von distal durch ein erstes Bauteil eines Schafts bis zu einer Montageposition, die proximal der für die fertiggestellte Komponente vorgesehenen Position relativ zu dem ersten Bauteil des Schafts liegt, einen Schritt des mechanischen Verbindens des proximalen Endes des ersten Bauteils der Übertragungseinrichtung mit einem zweiten Bauteil der Übertragungseinrichtung und einen Schritt des Bewegens des proximalen Endes des ersten Bauteils der Übertragungseinrichtung nach distal bis zu einer für die fertiggestellte Komponente vorgesehenen Position relativ zu dem ersten Bauteil des Schafts.

Das Verfahren ist insbesondere zum Herstellen einer Komponente, wie sie hier beschrieben ist oder für eine andere Komponente mit hier beschriebenen Merkmalen, Eigenschaften und Funktionen geeignet. Die Schritte des Hindurchführens, des Verbindens und des Bewegens werden insbesondere in dieser Reihenfolge ausgeführt. Das proximale Ende des ersten Bauteils der Übertragungseinrichtung wird mit dem zweiten Bauteil der Übertragungseinrichtung insbesondere dauerhaft bzw. nicht zerstörungsfrei lösbar verbunden. Das zweite Bauteil der Übertragungseinrichtung, mit dem das proximale Ende des ersten Bauteils der Übertragungseinrichtung verbunden ist, und das erste Bauteil des Schafts sind insbesondere so ausgeführt, dass eine Bewegung des zweiten Bauteils der Übertragungseinrichtung relativ zu dem ersten Bauteil des Schafts durch mechanischen Kontakt korrespondierender Anschlagflächen nach distal begrenzt ist.

Ein Verfahren, wie es hier beschrieben ist, umfasst insbesondere ferner einen Schritt des Einführens eines proximalen Endes eines Schaftrohrs in das erste Bauteil des Schafts bis zu einer Montageposition, die proximal der für die fertiggestellte Komponente vorgesehenen Position relativ zu dem ersten Bauteil des Schafts liegt, vor dem Verbinden des proximalen Endes des ersten Bauteils der Übertragungseinrichtung mit dem zweiten Bauteil der Übertragungseinrichtung, einen Schritt des Bewegens des proximalen Endes des Schaftrohrs relativ zu dem ersten Bauteil des Schafts nach distal bis zu der für die Verwendung der Komponente vorgesehenen Position des Schaftrohrs relativ zu dem ersten Bauteil des Schafts nach dem Verbinden des proximalen Endes des ersten Bauteils der Übertragungseinrichtung mit dem zweiten Bauteil der Übertragungseinrichtung, und einen Schritt des mechanischen Verbindens des proximalen Endes des Schaftrohrs mit dem ersten Bauteil des Schafts an der für die fertiggestellte Komponente vorgesehenen Position relativ zu dem ersten Bauteil des Schafts.

Das proximale Ende des Schaftrohrs wird mit dem ersten Bauteil des Schafts insbesondere dauerhaft bzw. nicht zerstörungsfrei lösbar verbunden.

Ein Verfahren, wie es hier beschrieben ist, umfasst insbesondere einen Schritt des Koppelns eines distalen Endes der Übertragungseinrichtung mit einem bewegbaren Teil eines Werkzeugs und einen Schritt des mechanischen Verbindens des Werkzeugs mit einem distalen Ende des Schafts.

Bei einem Verfahren, wie es hier beschrieben ist, werden insbesondere zumindest entweder der Schritt des Koppelns des distalen Endes der Übertragungseinrichtung mit einem bewegbaren Teil des Werkzeugs oder der Schritt des mechanischen Verbindens des Werkzeugs mit dem distalen Ende des Schafts zumindest entweder vor dem Schritt des Einführens des proximalen Endes des Schaftrohrs in das erste Bauteil des Schafts oder vor dem Schritt des Hindurchführens des proximalen Endes des ersten Bauteils der Übertragungseinrichtung von distal durch das erste Bauteil des Schafts ausgeführt.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische Darstellung einer Komponente eines medizinischen Instruments;
- Figur 3: eine schematische Darstellung eines Schnitts durch das proximale Ende einer Komponente eines medizinischen Instruments;
- Figur 4: eine weitere schematische Darstellung eines Schnitts durch das proximale Ende der Komponente aus Figur 3;
- Figur 5: eine schematische Darstellung eines weiteren Schnitts durch das proximale Ende der Komponente aus den Figuren 3 und 4;
- Figur 6: eine schematische Darstellung eines Schnitts durch eine weitere Komponente eines medizinischen Instruments;
- Figur 7: eine schematische Darstellung eines Schnitts durch das proximale Ende einer weiteren Komponente eines medizinischen Instruments;
- Figur 8: eine schematische Darstellung eines Schnitts durch Teile der Komponente aus Figur 7;
- Figur 9: eine weitere schematische Darstellung eines Schnitts durch die Teile aus Figur 8;
- Figur 10: eine weitere schematische Darstellung eines Schnitts durch die Teile aus den Figuren 8 und 9;
- Figur 11: eine schematische Darstellung eines Schnitts durch Teile der Komponente aus den Figuren 7 bis 10;
- Figur 12: eine weitere schematische Darstellung eines Schnitts durch die Teile aus Figur 11;
- Figur 13: eine weitere schematische Darstellung eines Schnitts durch Teile der Komponente aus Figur 7;
- Figur 14: eine schematische Darstellung eines Schnitts durch das proximale Ende einer weiteren Komponente eines medizinischen Instruments;
- Figur 15: eine weitere schematische Darstellung eines Schnitts durch Teile der Komponente aus Figur 14;
- Figur 16: eine weitere schematische Darstellung eines Schnitts durch Teile der Komponente aus Figur 14;
- Figur 17: ein schematisches Flussdiagramm eines Verfahrens zum Herstellen einer Komponente für ein medizinisches Instrument.

### Beschreibung der Ausführungsformen

Figur 1 zeigt eine schematische Darstellung eines medizinischen Instruments 10 mit einem Werkzeug 13 am distalen Ende einer Komponente 14. Das Werkzeug 13 ist in Figur 1 beispielhaft als Werkzeug zum Greifen, Halten, Quetschen, Schneider oder Stanzen von Gewebe zwischen einer feststehenden Branche und einer schwenkbaren Branche angedeutet.

Die Komponente 14 umfasst einen Schaft 15, der gerade oder gekrümmt, starr oder elastisch sein kann. Der Schaft 15 ist insbesondere flexibel und weist einen kreisförmigen Querschnitt mit einem Durchmesser von 2 mm oder weniger auf. Die Komponente 14 umfasst ferner eine Übertragungseinrichtung 16 zum Übertragen einer Kraft. Die Übertragungseinrichtung 16 ist im Inneren des Schafts 15 angeordnet und deshalb tatsächlich nicht sichtbar und in Figur 1 nur durch eine gestrichelte Linie angedeutet.

Das proximale Ende des Schafts 15 weist einen deutlich vergrößerten Querschnitt auf und ist mit einer Handhabungseinrichtung 18 mechanisch verbunden. Die Handhabungseinrichtung 18 weist ein manuell bewegbares Teil 19 auf. Das bewegbare Teil 19 ist mit dem proximalen Ende der Übertragungseinrichtung 16 derart gekoppelt, dass eine Schwenkbewegung des bewegbaren Teils 19 (insbesondere um eine Schwenkachse orthogonal zur Zeichenebene der Figur 1) relativ zu der übrigen Handhabungseinrichtung 18 mit einer Bewegung, insbesondere einer Translation der Übertragungseinrichtung 16 relativ zu dem Schaft 15 einhergeht. Das distale Ende der Übertragungseinrichtung 16 ist mit dem bewegbaren Teil des Werkzeugs 13 derart gekoppelt, dass eine Bewegung der Übertragungseinrichtung 16 relativ zu dem Schaft 15 mit einer Schwenkbewegung des bewegbaren Teils des Werkzeugs 13 relativ zu dem übrigen Werkzeug 13 einhergeht.

Die Komponente 14 ist von der Handhabungseinrichtung 18 zerstörungsfrei trennbar, wobei gleichzeitig die mechanische Kopplung des proximalen Endes der Übertragungseinrichtung 16 mit dem bewegbaren Teil 19 der Handhabungseinrichtung 18 beendet wird. Die Komponente 14 ist also dazu ausgebildet, um zusammen mit der Handhabungseinrichtung 18 das medizinische Instrument 10 zu bilden.

Figur 2 zeigt eine schematische Darstellung einer weiteren Komponente 14 für ein medizinisches Instrument. Die Komponente 14 ähnelt in einigen Merkmalen, Eigenschaften und Funktionen der anhand der Figur 1 dargestellten Komponente. Insbesondere können das proximale Ende des Schafts 15 mit einer Handhabungseinrichtung zerstörungsfrei lösbar oder dauerhaft bzw. nicht zerstörungsfrei lösbar mechanisch verbunden und das proximale Ende der Übertragungseinrichtung 16 mit einem bewegbaren Teil der Handhabungseinrichtung zerstörungsfrei lösbar oder dauerhaft bzw. nicht zerstörungsfrei lösbar gekoppelt werden.

Die in Figur 2 dargestellte Komponente 14 unterscheidet sich von der anhand der Figur 1 dargestellten Komponente insbesondere dadurch, dass sie am distalen Ende kein Werkzeug aufweist, sondern lediglich mit einem Werkzeug 13 verbunden und gekoppelt werden kann. Das Werkzeug 13 ist deshalb in gestrichelten Linien angedeutet. Das distale Ende des Schafts 15 der Komponente 14 kann zur dauerhaften bzw. nicht zerstörungsfrei lösbaren oder zur zerstörungsfrei lösbaren mechanischen Verbindung mit einem feststehenden Teil des Werkzeugs 13 vorgesehen und ausgebildet sein. Das distale Ende der Übertragungseinrichtung 16 kann zur dauerhaften bzw. nicht zerstörungsfrei lösbaren oder zur zerstörungsfrei lösbaren mechanischen Kopplung mit einem bewegbaren Teil des Werkzeugs 13 vorgesehen und ausgebildet sein.

Figur 3 zeigt eine schematische Darstellung eines Schnitts durch ein proximales Ende einer Komponente 14 zur Bildung eines medizinischen Instruments. Die in Figur 3 dargestellte Komponente 14 ähnelt in einigen - insbesondere in den nicht anhand der Figuren 3 bis 5 dargestellten - Merkmalen, Eigenschaften und Funktionen den anhand der Figur 1 und 2 dargestellten Komponenten.

Bei der in Figur 3 gezeigten Komponente 14 umfasst eine Übertragungseinrichtung (vgl. Bezugszeichen 16 aus den Figuren 1 und 2) von distal (in Figur 3: links) nach proximal (in Figur 3: rechts) einen Draht 20 als langen dünnen Abschnitt der Übertragungseinrichtung, ein erstes Stabbauteil 30 und ein zweites Stabbauteil 40. Bei der in Figur 3 gezeigten Komponente 14 umfasst der Schaft (vgl. Bezugszeichen 15 aus den Figuren 1 und 2) von distal nach proximal ein langes dünnes Schaftrohr 50, ein erstes Hülsenbauteil 60, ein zweites Hülsenbauteil 70 und ein drittes Hülsenbauteil 80. Das proximale Ende des Schaftrohrs 50, das erste Hülsenbauteil 60 und das distale Ende des zweiten Hülsenbauteils 70 sind ferner von einem Anschlussbauteil 90 umgeben.

Der Draht 20 weist die Gestalt eines langen dünnen Zylinders, insbesondere eines Kreiszylinders auf. Der Draht 20 ist zur Übertragung einer Kraft zu einem Werkzeug am distalen Ende der Komponente 14 vorgesehen und ausgebildet. Der Draht 20 ist insbesondere aus chirurgischem Stahl, Federstahl oder einem anderen Metall gebildet. Alternativ kann der Draht aus Kunststoff gefertigt sein oder einen Kunststoff enthalten.

Das proximale Ende 23 des Drahts 20 ist in einer ersten Längsbohrung 32 am distalen Ende des ersten Stabbauteils 30 angeordnet und mit diesem beispielsweise durch Schweißen, Löten, eine Klebung oder auf andere stoff-, kraft- und / oder formschlüssige Weise mechanisch starr verbunden. Das erste Stabbauteil 30 weist an seinem proximalen Ende eine zweite Längsbohrung 34 auf, in der ein Zapfen 43 am distalen Ende des zweiten Stabbauteils angeordnet ist. Der Zapfen 43 am distalen Ende des zweiten Stabbauteils ist mit der zweiten Längsbohrung 34 am proximalen Ende des ersten Stabbauteils 30 beispielsweise durch eine Schraubverbindung zwischen korrespondierenden Innen- und Außengewinden und / oder auf andere Weise mechanisch starr verbunden. Das zweite Stabbauteil 40 weist an seinem proximalen Ende eine Kopplungskugel 49 zur lösbaren mechanischen Kopplung mit einem bewegbaren Teil 19 einer Handhabungseinrichtung 18 (vgl. Figur 1) auf.

Das erste Stabbauteil 30 weist ferner einen Kragen 35 auf. Der Kragen ist bei dem in Figur 3 gezeigten Beispiel an oder nahe dem proximalen Ende des ersten Stabbauteils 30 angeordnet. Der Kragen 35 ragt radial nach außen und umgibt das übrige erste Stabbauteil 30 ringförmig. An dem Kragen 35 sind eine nach proximal orientierte, ebene und ringförmige Anschlagfläche 36 und eine nach distal orientierte, ebene und ringförmige Anschlagfläche 37 vorgesehen.

Das Schaftrohr 50 weist einen Kanal 52 auf. Der Querschnitt des Kanals 52 in dem Schaftrohr 50 und der Querschnitt des Drahts 20 sind so gewählt, dass der Draht 20 spiel- und vor allem reibungsarm in dem Kanal 52 in dem Schaftrohr 50 geführt ist. Das Schaftrohr 50 weist beispielsweise einen kreisringförmigen Querschnitt und einen Außendurchmesser von 2 mm oder weniger auf. Der Draht 20 weist beispielsweise einen kreisförmigen Querschnitt und einen Außendurchmesser von wenigen Zehntelmillimetern auf.

Das erste Hülsenbauteil 60 weist einen im Wesentlichen kreiszylindrischen Kanal auf, in dem das proximale Ende 56 des Schaftrohrs 50 angeordnet ist, und der durch einen nach radial innen ragenden Kragen 66 am proximalen Ende des ersten Hülsenbauteils 60 verengt wird. Das proximale Ende 56 des Schaftrohrs 50 liegt an einer nach distal orientierten, kreisringförmigen Fläche an dem Kragen 66 an. Der Kragen 66 verengt den durch das erste Hülsenbauteil 60 umschlossenen Kanal auf einem Querschnitt, der näherungsweise dem Querschnitt des Kanals 52 in dem Schaftrohr 50 entspricht oder größer ist. Somit kann der Draht 20 auch relativ zu dem ersten Hülsenbauteil 60 reibungsarm bewegt werden.

Das zweite Hülsenbauteil 70 umschließt einen Kanal 73, der sich vom distalen Ende bis zum proximalen Ende des zweiten Hülsenbauteils 70 erstreckt. Der Querschnitt des Kanals 73 in dem zweiten Hülsenbauteil 70 und der Querschnitt des ersten Stabbauteils 30 sind so gewählt, dass das erste Stabbauteil 30 in dem Kanal 73 in dem zweiten Hülsenbauteil 70 spiel- und reibungsarm geführt ist.

Der Kanal 73 in dem zweiten Hülsenbauteil 70 ist an einem Ort durch eine nach radial au-ßen sich erstreckende Nut, in der ein O-Ring 74 angeordnet ist, erweitert. Die Nut, der O-Ring 74 und die äußere Kontur des Querschnitts des ersten Stabbauteils 30 sind so gewählt, dass der O-Ring 74 an der äußeren Mantelfläche des ersten Stabbauteils 30 jederzeit anliegt. Der O-Ring 74 kann auf diese Weise einen Übertritt eines Fluids von distal nach proximal unterbinden oder zumindest behindern. Ferner kann der O-Ring 74 eine Bewegung der Übertragungseinrichtung 20, 30, 40 relativ zu dem Schaft 50, 60, 70, 80 bremsen. Um die Erzeugung der Nut für den O-Ring 74 zu vereinfachen, ist der Querschnitt des Kanals 73 in dem zweiten Hülsenbauteil 70 distal der Nut größer als proximal der Nut.

Der Querschnitt des Kanals 73 in dem zweiten Hülsenbauteil 70 ist nahe dem distalen Ende des zweiten Hülsenbauteils 70 stufenförmig erweitert, um das erste Hülsenbauteil 60 aufzunehmen, und die Position des ersten Hülsenbauteils 60 relativ zu dem zweiten Hülsenbauteil 70 formschlüssig zu definieren. Das erste Hülsenbauteil 60 ist im distalen Ende des Kanals 73 in dem zweiten Hülsenbauteil 70 insbesondere mittels einer Schraubverbindung und / oder auf andere form-, kraft- und / oder stoffschlüssige Weise gefügt.

Das dritte Hülsenbauteil 80 weist einen Kanal 83 auf, in dem vor allem das zweite Stabbauteil 40 angeordnet ist. Der Querschnitt des Kanals 83 in dem dritten Hülsenbauteil 80 und die äußere Kontur des Querschnitts des zweiten Stabbauteils 40 sind so gewählt, dass das zweite Stabbauteil 40 spiel- und vor allem reibungsarm in dem Kanal 83 in dem dritten Hülsenbauteil 80 geführt ist.

Das proximale Ende des zweiten Hülsenbauteils 70 und das distale Ende des dritten Hülsenbauteils 80 sind starr miteinander verbunden. Bei dem dargestellten Beispiel sind sowohl das proximale Ende des zweiten Hülsenbauteils 70 als auch das distale Ende des dritten Hülsenbauteils 80 jeweils rohrförmig, jedoch mit unterschiedlichen Durchmessern ausgebildet. Das rohrförmige proximale Ende des zweiten Hülsenbauteils 70 steckt in dem rohrförmigen distalen Ende des dritten Hülsenbauteils 80 und ist mit diesem form-, kraft- und / oder stoffschlüssig gefügt.

Eine nach distal stufenförmige Erweiterung des Kanals 83 in dem dritten Hülsenbauteil 80 bildet eine ringförmige Ausnehmung 85 in Gestalt einer von dem Kanal 83 in dem dritten Hülsenbauteil 80 aus nach radial außen ragenden, flachen Nut. In der Ausnehmung 85 ist der Kragen 35 an dem ersten Stabbauteil 30 angeordnet. Der Querschnitt der Ausnehmung 85 und die äußere Kontur des Kragens 35 an dem ersten Stabbauteil sind so gewählt, dass der Kragen 35 in der Ausnehmung 85 reibungsarm bewegt werden kann.

Der nach distal orientierte, ringförmige und ebene Bereich der Oberfläche der Ausnehmung 85 bildet eine nach distal orientierte Anschlagfläche 86. Mechanischer Kontakt zwischen der nach proximal orientierten Anschlagfläche 36 an dem Kragen 35 an dem ersten Stabbauteil 30 und der nach distal orientierten Anschlagfläche 86 in der Ausnehmung 85 begrenzt eine Bewegung der Übertragungseinrichtung 20, 30, 40 relativ zu dem Schaft 50, 60, 70, 80 nach proximal.

Die nach proximal orientierte ringförmige und ebene Randfläche des zweiten Hülsenbauteils 70 bildet den distalen Rand der Ausnehmung 85 und eine nach proximal orientierte Anschlagfläche des Schafts 50, 60, 70, 80. Mechanischer Kontakt zwischen der nach distal orientierten Anschlagfläche 37 an dem Kragen 35 an dem ersten Stabbauteil 30 und der nach proximal orientierten Anschlagfläche 78 an dem Schaft 50, 60, 70, 80 begrenzt eine Bewegung der Übertragungseinrichtung 20, 30, 40 relativ zu dem Schaft 50, 60, 70, 80 nach distal.

Das Anschlussbauteil 90 weist einen Spülanschluss 95 auf, der insbesondere dem Luer-Lock-System entspricht. Der Spülanschluss 95 fluchtet mit einer Spülöffnung 59 in dem Schaftrohr 50 und ermöglicht eine Zufuhr eines Spülfluids in den Zwischenraum zwischen dem Draht 20 und dem Schaftrohr 50 zur Reinigung der Komponente.

Figur 4 zeigt eine weitere schematische Darstellung eines Schnitts durch das proximale Ende der anhand der Figur 3 dargestellten Komponente. Die Schnittebene der Figur 4 entspricht der Schnittebene der Figur 3.

In Figur 4 ist eine Situation bzw. Konfiguration dargestellt, bei der die nach proximal orientierte Anschlagfläche 36 an dem Kragen 35 an der Übertragungseinrichtung 20, 30, 40 an der nach distal orientierten Anschlagfläche 86 an dem Schaft 50, 60, 70, 80 anliegt. Die in Figur 4 dargestellte Position der Übertragungseinrichtung 20, 30, 40 relativ zu dem Schaft 50, 60, 70, 80 ist deshalb die äußerst proximale erreichbare Position. Wenn ein in den Figuren 3 und 4 nicht dargestelltes Werkzeug am distalen Ende der Komponente 14 eine oder zwei bewegbare Branchen bzw. Maulteile aufweist und zum Greifen, Halten, Quetschen, Schneiden oder Stanzen vorgesehen ist, liegen die Maulteile des Werkzeugs bei der in Figur 4 gezeigten Situation ganz aneinander an und werden gegeneinander gepresst.

Die Komponente 14, insbesondere die Positionen der nach proximal orientierten Anschlagfläche 36 an dem Kragen 35 an der Übertragungseinrichtung 20, 30, 40 und der nach distal orientierten Anschlagfläche 86 an dem Schaft 50, 60, 70, 80 sowie die elastischen Eigenschaften des Drahts 20 sind so gewählt, dass bei der in Figur 4 gezeigten Situation der Draht 20 lediglich elastisch, jedoch nicht plastisch verformt wird und auch das Schaftrohr 50 und das Werkzeug durch die dabei wirkenden Kräfte nicht zerstört oder geschädigt werden. Dies gilt insbesondere selbst dann, wenn das Werkzeug im ganz geöffneten Zustand blockiert ist, beispielsweise durch einen harten bzw. nicht nachgebenden Gegenstand zwischen den Maulteilen.

Figur 5 zeigt eine Darstellung eines weiteren Schnitts durch die anhand der Figuren 3 und 4 dargestellte Komponente. Die Schnittebene der Figur 5 ist orthogonal zur Schnittebene der Figuren 3 und 4 und zu der Längs- und Symmetrieachse der Übertragungseinrichtung 20, 30, 40. Die Schnittebene der Figur 5 schneidet die Ausnehmung 85 und liegt proximal des Kragens 35 an der Übertragungseinrichtung 20, 30, 40. Figur 5 zeigt deshalb eine Situation ähnlich der in Figur 3 gezeigten.

In Figur 5 ist erkennbar, dass der Kragen 35 die Stabbauteile 30, 40 kreisringförmig umschließt, und dass die Ausnehmung 85 ebenfalls kreisringförmig ausgebildet ist.

Figur 6 zeigt eine schematische Darstellung eines Schnitts durch eine weitere Komponente, die der anhand der Figuren 3 bis 5 dargestellten Komponente 14 in einigen Merkmalen, Eigenschaften und Funktionen ähnelt. Die Schnittebene der Figur 6 entspricht der Schnittebene der Figur 5. Nachfolgend sind insbesondere Merkmale, Eigenschaften und Funktionen beschrieben, in denen die in Figur 6 gezeigte Komponente sich von der anhand der Figuren 3 bis 5 dargestellten Komponente unterscheidet. Ein Längsschnitt durch die in Figur 6 gezeigte Komponente, wie er in den Figuren 3 und 4 gezeigt ist, würde sich von den in den Figuren 3 und 4 gezeigten Schnitten nicht unterscheiden. Deshalb wird nachfolgend auch auf die Figuren 3 und 4 Bezug genommen.

Die in Figur 6 gezeigte Komponente 14 weist keine ringförmige, den Kanal 30, 40 in dem Schaft 50, 60, 70, 80 erweiternde Ausnehmung, sondern eine oder mehrere nischenförmige Ausnehmungen 85 auf. Bei dem dargestellten Beispiel liegen zwei Ausnehmungen 85 einander symmetrisch gegenüber. Die Ausnehmungen 85 erweitern nischenförmig den Kanal 73, 83 in dem Schaft 50, 60, 70, 80, in dem die Übertragungseinrichtung 20, 30, 40 angeordnet ist. Das dritte Hülsenbauteil 80 weist bei der in Figur 6 gezeigten Komponente insbesondere die gleiche Gestalt auf wie bei der anhand der Figuren 3 bis 5 dargestellten Komponente. Das zweite Hülsenbauteil 70 weist jedoch keine kreisringförmige proximale Randfläche 78, sondern eine gestufte Randfläche auf. Der proximale Rand des zweiten Hülsenbauteils 70 liegt insbesondere an der nach distal orientierten kreisringförmigen Anschlagfläche 86 an dem dritten Hülsenbauteil 80 an, ist jedoch durch zwei einander gegenüberliegende Schlitze unterbrochen, die die Ausnehmungen 85 bilden. Die distalen Enden dieser Schlitze bilden nach proximal orientierte Anschlagflächen 78 (vgl. Figur 3).

Bei der in Figur 6 gezeigten Komponente weist die Übertragungseinrichtung 20, 30, 40 keinen ringförmigen Kragen, sondern zwei einander gegenüberliegende Vorsprünge oder Nasen oder Zapfen 35 auf. Die Nasen 35 sind in den Ausnehmungen 85 angeordnet.

Ähnlich wie bei der anhand der Figuren 3 bis 5 dargestellten Komponente begrenzt auch bei der in Figur 6 gezeigten Komponente ein mechanischer Kontakt der Nasen 35 bzw. von Anschlagflächen an den Nasen 35 an der Übertragungseinrichtung 20, 30, 40 mit Anschlagflächen an dem Schaft 50, 60, 70, 80 eine Bewegung der Übertragungseinrichtung 20, 30, 40 relativ zu dem Schaft 50, 60, 70, 80 sowohl nach proximal als auch nach distal.

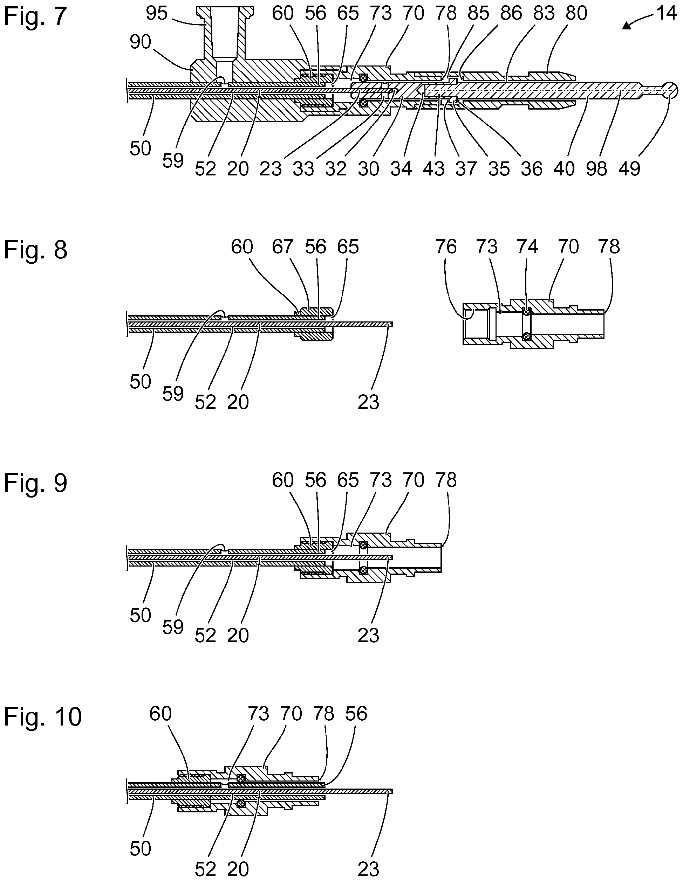

Figur 7 zeigt eine schematische Darstellung eines Schnitts durch eine weitere Komponente 14, die in einigen Merkmalen, Eigenschaften und Funktionen den anhand der Figuren 3 bis 6 dargestellten Komponenten entspricht. Die in Figur 7 gezeigte Schnittebene entspricht der Schnittebene der Figuren 3 und 4. Nachfolgend sind insbesondere Merkmale, Eigenschaften und Funktionen der Komponente 14 beschrieben, in denen diese sich von den anhand der Figuren 3 bis 6 dargestellten Komponenten unterscheidet.

Die in Figur 7 gezeigte Komponente 14 kann einen ringförmigen Kragen 35 an der Übertragungseinrichtung 20, 30, 40 in einer ringförmigen Ausnehmung 85 in dem Schaft 50, 60, 70, 80 aufweisen, ähnlich wie die anhand der Figuren 3 bis 5 dargestellte Komponente. Alternativ kann die in Figur 7 gezeigte Komponente 14 eine oder mehrere Nasen an der Übertragungseinrichtung 20, 30, 40 aufweisen, die in jeweils einer zugeordneten Ausnehmung 85 in dem Schaft 50, 60, 70, 80 angeordnet sind, ähnlich wie bei der anhand der Figur 6 dargestellten Komponente.

Die in Figur 7 gezeigte Komponente 14 unterscheidet sich von den anhand der Figuren 3 bis 6 dargestellten Komponenten insbesondere dadurch, dass das erste Hülsenbauteil 60 keinen nach innen ragenden Kragen am proximalen Ende aufweist. Der Kanal 65 in dem ersten Hülsenbauteil 60 ist vielmehr durchgehend zylindrisch, insbesondere kreiszylindrisch. Dies ermöglicht eine Fertigung der Komponente 14, wie sie anhand der Figuren 8 bis 13 dargestellt ist.

Figur 8 zeigt eine schematische Darstellung eines Schnitts durch Teile der anhand der Figur 7 dargestellten Komponente 14 während deren Fertigung. Die Schnittebene der Figur 8 entspricht den Schnittebenen der Figuren 3, 4 und 7.

In Figur 8 ist eine Situation während der Fertigung der Komponente gezeigt, bei der bereits der Draht 20 in dem Kanal 52 in dem Schaftrohr 50 angeordnet ist. Das proximale Ende 56 des Schaftrohrs 50 ist in den Kanal 65 in dem ersten Hülsenbauteil 60 eingeführt. Das Schaftrohr 50 ist jedoch noch nicht starr mit dem ersten Hülsenbauteil 60 verbunden, sondern kann, wie nachfolgend anhand der Figuren 10 bis 12 beschrieben, relativ zu dem ersten Hülsenbauteil 60 verschoben werden. Das erste Hülsenbauteil 60 weist ein Außengewinde 67 auf, das zur form- und kraftschlüssigen Fügung mit einem korrespondierenden Innengewinde 76 am distalen Ende des zweiten Hülsenbauteils 70 vorgesehen und ausgebildet ist.

Figur 9 zeigt eine weitere schematische Darstellung eines Schnitts durch die in Figur 8 gezeigten Teile. Die Schnittebene der Figur 9 entspricht den Schnittebenen der Figuren 3, 4, 7 und 8.

Bei der in Figur 9 gezeigten Situation ist das erste Hülsenbauteil 60 durch eine Schraubverbindung zwischen seinem Außengewinde 67 (vgl. Figur 8) mit dem Innengewinde 76 an dem zweiten Hülsenbauteil 70 mit dem distalen Ende des zweiten Hülsenbauteils 70 verbunden. Alternativ oder zusätzlich können das erste Hülsenbauteil 60 und das zweite Hülsenbauteil 70 auf andere Weise form-, kraft- und / oder stoffschlüssig miteinander verbunden sein.

Figur 10 zeigt eine weitere schematische Darstellung eines Schnitts durch die in den Figuren 8 und 9 gezeigten Teile. Die Schnittebene der Figur 10 entspricht den Schnittebenen der Figuren 3, 4 und 7 bis 9.

Bei der in Figur 10 gezeigten Situation ist die Einheit aus dem ersten Hülsenbauteil 60 und dem zweiten Hülsenbauteil 70 relativ zu dem Draht 20 und dem Schaftrohr 50 nach distal verschoben, so dass das proximale Ende 23 des Drahts 20 und optional auch wie in Figur 10 angedeutet das proximale Ende 56 des Schaftrohrs 50 nach proximal aus dem Kanal 73 in dem zweiten Hülsenbauteil 70 herausragen.

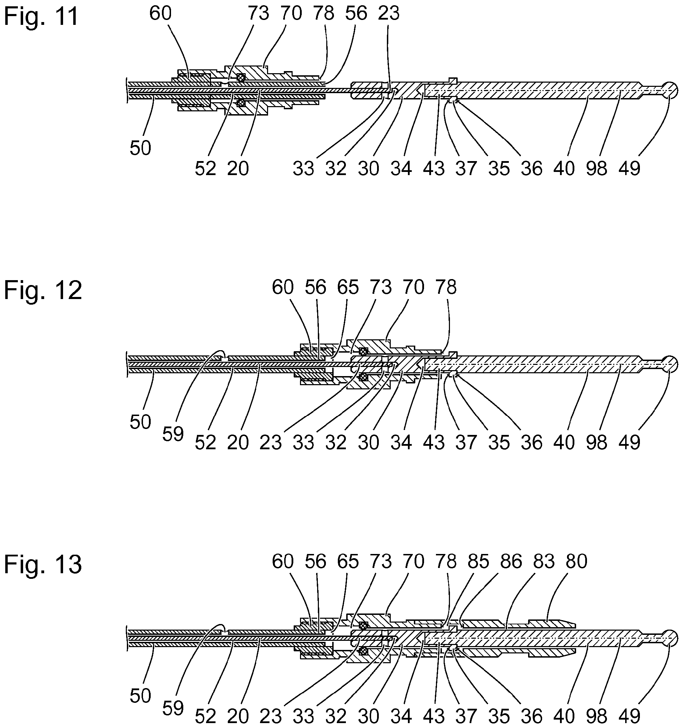

Figur 11 zeigt eine weitere schematische Darstellung eines Schnitts durch Teile der anhand der Figur 7 dargestellten Komponente 14 während deren Fertigung. Die Schnittebene der Figur 11 entspricht den Schnittebenen der Figuren 3, 4 und 7 bis 10.

Bei der in Figur 11 gezeigten Situation ist das aus dem zweiten Hülsenbauteil 70 herausragende proximale Ende 23 des Drahts 20 in die erste Längsbohrung 32 an dem distalen Ende des ersten Stabbauteils 30 eingeführt. Bei dem dargestellten Beispiel ist im Bereich der ersten Längsbohrung 32 eine Querbohrung 33 in dem ersten Stabbauteil 30 vorgesehen, um ein stoffschlüssiges Fügen des proximalen Endes 23 des Drahts 20 mit dem distalen Ende des ersten Stabbauteils 30 zu ermöglichen oder zu vereinfachen. Insbesondere wird das proximale Ende 23 des Drahts 20 durch Laserschweißen im Bereich der Querbohrung 33 mit dem ersten Stabbauteil 30 stoffschlüssig gefügt. Alternativ oder zusätzlich können das proximale Ende 23 des Drahts 20 und das erste Stabbauteil 30 auf andere Weise miteinander form-, kraft- und / oder stoffschlüssig verbunden werden.

Eine Querbohrung 33 in dem ersten Stabbauteil 30, wie sie in den Figuren 7 und 11 gezeigt ist, kann auch bei den anhand der Figuren 3 bis 6 dargestellten Komponenten vorgesehen sein.

Figur 12 zeigt eine weitere schematische Darstellung eines Schnitts durch die in Figur 11 gezeigten Teile. Die Schnittebene der Figur 12 entspricht den Schnittebenen der Figuren 3, 4 und 7 bis 11.

Bei der in Figur 12 gezeigten Situation ist die Einheit aus dem ersten Hülsenbauteil 60 und dem zweiten Hülsenbauteil 70 relativ zu dem Schaftrohr 50 und auch relativ zu der Übertragungseinrichtung 20, 30, 40 wieder nach proximal verschoben. Die in Figur 12 gezeigte Position der Einheit aus dem ersten Hülsenbauteil 60 und dem zweiten Hülsenbauteil 70 relativ zu dem proximalen Ende 56 des Schaftrohrs 50 entspricht der endgültigen bzw. für die fertige Komponente vorgesehenen Position. In dieser Position wird das proximale Ende 56 des Schaftrohrs 50 mit dem ersten Hülsenbauteil 60 mechanisch starr verbunden, beispielsweise durch eine umlaufende Laserschweißnaht oder auf andere form-, kraft- und / oder stoffschlüssige Weise.

Figur 13 zeigt eine weitere schematische Darstellung eines Schnitts durch Teile der anhand der Figur 7 dargestellten Komponente 14 während deren Fertigung. Die Schnittebene der Figur 13 entspricht den Schnittebenen der Figuren 3, 4 und 7 bis 12.

Bei der in Figur 13 gezeigten Situation ist das dritte Hülsenbauteil 80 in der vorgesehenen Weise an das zweite Hülsenbauteil 70 angesetzt und kann mit diesem form-, kraft- und / oder stoffschlüssig verbunden sein oder werden. Zur Fertigstellung der anhand der Figur 7 dargestellten Komponente muss nun nur noch das Anschlussbauteil 90 über das Schaftrohr 50, das erste Hülsenbauteil 60 und das distale Ende des zweiten Hülsenbauteils 70 gestülpt und mit diesem form-, kraft- und / oder stoffschlüssig gefügt werden.

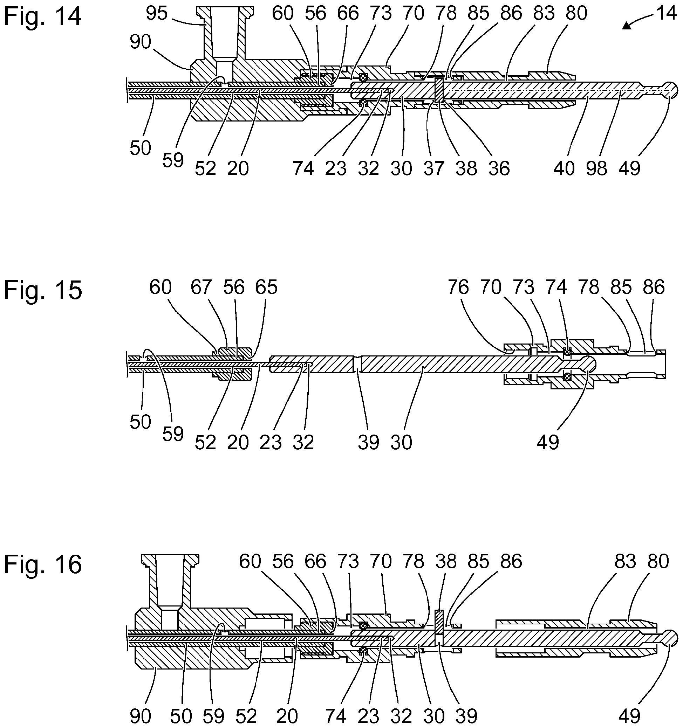

Figur 14 zeigt eine schematische Darstellung eines Schnitts durch das proximale Ende einer weiteren Komponente 14, die in einigen Merkmalen, Eigenschaften und Funktionen den anhand der Figuren 3 bis 13 dargestellten Komponenten ähnelt. Die Schnittebene der Figur 14 entspricht den Schnittebenen der Figuren 3, 4 und 7 bis 13. Nachfolgend sind insbesondere Merkmale und Eigenschaften der Komponente 14 beschrieben, in denen diese sich von den anhand der Figuren 3 bis 13 dargestellten Komponenten unterscheidet. Die in Figur 14 gezeigte Komponente 14 unterscheidet sich von den anhand der Figuren 3 bis 13 dargestellten Komponenten insbesondere dadurch, dass lediglich ein Stabbauteil 30 vorgesehen ist, das die Funktionen beider Stabbauteile 30, 40 der anhand der Figuren 3 bis 13 dargestellten Komponenten vereint. Das Stabbauteil 30 umfasst insbesondere an seinem distalen Ende eine Längsbohrung 32, in der das proximale Ende 23 des Drahts 20 angeordnet und befestigt ist, und an seinem proximalen Ende eine Kopplungskugel 49 zur lösbaren mechanischen Kopplung mit einem bewegbaren Teil 19 einer Handhabungseinrichtung 18 (vgl. Figur 1).

Auch bei den anhand der Figuren 3 bis 13 dargestellten Komponenten kann anstelle zweier miteinander verbundener Stabbauteile jeweils ein einziges Stabbauteil 30 vorgesehen sein, das wie bei der in Figur 14 gezeigten Komponente 14 alle Funktionen der Stabbauteile in sich vereint.

Die in Figur 14 gezeigte Komponente 14 unterscheidet sich von den anhand der Figuren 3 bis 13 dargestellten Komponenten ferner dadurch, dass weder ein ringförmiger Kragen noch eine oder mehrere Nasen an dem Stabbauteil 30 vorgesehen sind. Vielmehr ist ein Stift 38 in einer entsprechenden Querbohrung in dem Stabbauteil 30 vorgesehen. Beide Enden des Stifts 38 stehen gegenüber der äußeren Kontur des Stabbauteils 30 über und ragen in je eine längliche Ausnehmung 85 in dem Schaft 50, 60, 70, 80 hinein. Nach proximal orientierte Anschlagflächen 36 und nach distal orientierte Anschlagflächen 37 der Übertragungseinrichtung 20, 30, 40 werden durch entsprechende Teile der Oberflächen der Enden des Stifts 38 gebildet.

Die Ausnehmungen 85 werden durch Schlitze in dem zweiten Hülsenbauteil 70 gebildet, die nach außen durch einen rohrförmigen Abschnitt des dritten Hülsenbauteils 80 abgeschlossen werden. Randbereiche der die Ausnehmungen 85 bildenden Schlitze in dem zweiten Hülsenbauteil 70 bilden die nach proximal orientierte Anschlagfläche 78 und die nach distal orientierte Anschlagfläche 86.

Mechanischer Kontakt zwischen den nach proximal orientierten Anschlagflächen 36 an dem Stift 38 und den nach distal orientierten Anschlagflächen 86 an den proximalen Enden der Ausnehmungen 85 begrenzt die Bewegung der Übertragungseinrichtung 20, 30 relativ zu dem Schaft 50, 60, 70, 80 nach proximal. Mechanischer Kontakt zwischen den nach distal orientierten Anschlagflächen 37 an den Enden des Stifts 38 und den nach proximal orientierten Anschlagflächen 78 an den distalen Enden der Ausnehmungen 85 begrenzen die Bewegung der Übertragungseinrichtung 20, 30 relativ zu dem Schaft 50, 60, 70, 80 nach distal.

Figur 15 zeigt eine schematische Darstellung eines Schnitts durch Teile der anhand der Figur 14 dargestellten Komponente. Die Schnittebene der Figur 15 entspricht den Schnittebenen der Figuren 3, 4 und 7 bis 14.

In Figur 15 ist eine Situation gezeigt, bei der das erste Hülsenbauteil 60 bereits mit dem proximalen Ende 56 des Schaftrohrs 50 mechanisch starr verbunden sein kann. Ferner ist das proximale Ende 23 des Drahts 20 bereits mit dem distalen Ende des Stabbauteils 30 mechanisch starr verbunden, nämlich in die Längsbohrung 32 am distalen Ende des Stabbauteils 30 gefügt. Das proximale Ende des Stabbauteils 30 ist bereits teilweise in den Kanal 73 in dem zweiten Hülsenbauteil 70 eingeführt. In dem zweiten Hülsenbauteil 70 sind einander gegenüberliegende Schlitze, die die Ausnehmungen 85 bilden, und deren proximale bzw. distale Enden die nach distal orientierten Anschlagflächen 86 bzw. die nach proximal orientierten Anschlagflächen 78 bilden, erkennbar.

Ausgehend von der in Figur 15 gezeigten Situation kann das zweite Hülsenbauteil 70 relativ zu den anderen dargestellten Bauteilen nach distal verschoben und mit dem ersten Hülsenbauteil 60 verbunden werden, beispielsweise durch Schraubverbindung zwischen einem Außengewinde 67 an dem ersten Hülsenbauteil 60 und einem Innengewinde 76 an dem zweiten Hülsenbauteil 70.

Figur 16 zeigt eine weitere schematische Darstellung eines Schnitts durch Teile der anhand der Figur 14 dargestellten Komponente 14 während deren Fertigung. Die Schnittebene der Figur 16 entspricht den Schnittebenen der Figuren 3, 4 und 7 bis 15.

Die in Figur 16 gezeigte Situation unterscheidet sich von der in Figur 15 gezeigten Situation dadurch, dass das erste Hülsenbauteil 60 starr und insbesondere dauerhaft mit dem zweiten Hülsenbauteil 70 mechanisch starr verbunden ist. Der Stift 38 ist bereits teilweise durch einen der beiden Schlitze an dem zweiten Hülsenbauteil 70, die die beiden Ausnehmungen 85 bilden, in eine korrespondierende Querbohrung 39 in dem Stabbauteil 30 eingeführt. Wenn der Stift 38 relativ zu dem Stabbauteil 30 seine vorgesehene und in Figur 14 gezeigte Position erreicht hat, kann das in Figur 16 bereits über das proximale Ende des Stabbauteils 30 gestülpt gezeigte dritte Hülsenbauteil 80 an das proximale Ende des zweiten Hülsenbauteils 70 angesetzt und mit diesem mechanisch starr und insbesondere dauerhaft verbunden werden.

Ferner kann bereits davor oder danach das Anschlussbauteil 90 von distal her über das erste Hülsenbauteil 60 und das distale Ende des zweiten Hülsenbauteils 70 gestülpt werden. Eine mechanisch starre Verbindung zwischen dem Anschlussbauteil 90 und dem zweiten Hülsenbauteil 70 wird bei den anhand der Figuren 3 bis 16 dargestellten Beispielen jeweils durch Eingriff eines nach innen ragenden Kragens am proximalen Ende des Anschlussbauteils 90 in eine korrespondierende umlaufende Nut an dem zweiten Hülsenbauteil 70 gebildet. Alternativ oder zusätzlich kann das Anschlussbauteil 90 bei allen anhand der Figuren 3 bis 16 gezeigten Komponenten 14 form-, kraft- und / oder stoffschlüssig mit dem Schaftrohr 50 und / oder mit dem zweiten Hülsenbauteil 70 gefügt werden.

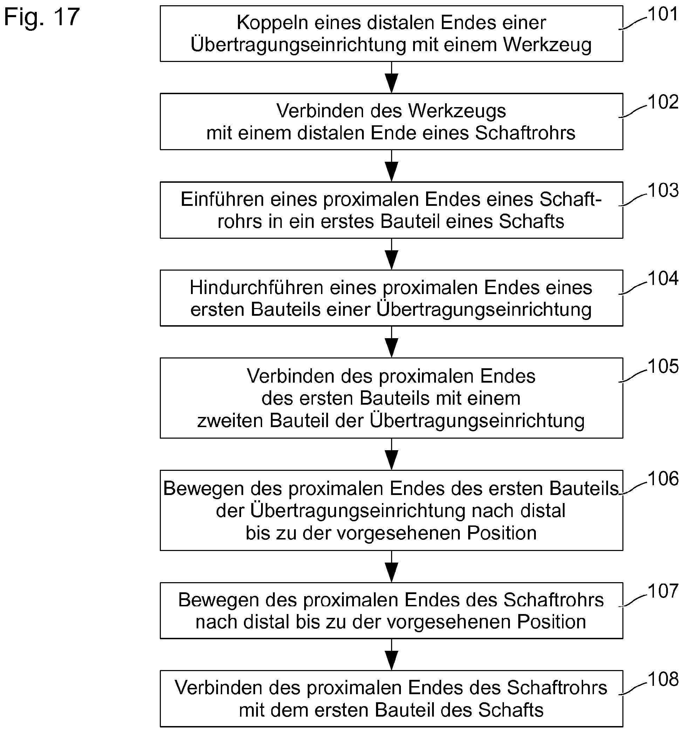

Figur 17 zeigt ein schematisches Flussdiagramm eines Verfahrens zum Herstellen einer Komponente für ein medizinisches Instrument, insbesondere einer der anhand der Figuren 3 bis 16 dargestellten Komponenten. Das Verfahren ist auch geeignet zum Herstellen einer Komponente mit Merkmalen, Eigenschaften und Funktionen, die von den anhand der Figuren 1 bis 16 dargestellten abweichen. Trotzdem werden nachfolgend Bezugszeichen aus den Figuren 1 bis 16 beispielhaft verwendet, um das Verständnis zu vereinfachen.

Bei einem ersten Schritt 101 wird ein distales Ende einer Übertragungseinrichtung 16, 20, 30, 40 mit einem bewegbaren Teil eines Werkzeugs 13 gekoppelt. Das Werkzeug 13 ist insbesondere zum Greifen, Halten, Quetschen, Schneider oder Stanzen vorgesehen und weist beispielsweise eine feststehende Branche bzw. ein feststehendes Maulteil und eine relativ zu der feststehenden Branche schwenkbare Branche auf. Die schwenkbare Branche kann unmittelbar durch ein Gelenk oder mittelbar über einen Pleuel mit dem distalen Ende der Übertragungseinrichtung 16, 20, 30, 40 gekoppelt werden. Alternativ kann die schwenkbare Branche auf andere Weise derart mit dem distalen Ende der Übertragungsrichtung 16, 20, 30, 40 gekoppelt werden, dass eine Translation der Übertragungseinrichtung 16, 20, 30, 40 mit einer Schwenkbewegung der schwenkbaren Branche einhergeht.

Bei einem zweiten Schritt 102 wird das Werkzeug 13 mit dem distalen Ende des Schafts 15, 50, 60, 70, 80 verbunden. Bei dem zweiten Schritt 102 wird das Werkzeug insbesondere dauerhaft bzw. nicht zerstörungsfrei lösbar mit dem distalen Ende des Schafts 15, 50, 60, 70, 80 verbunden.

Bei einem dritten Schritt 103 wird ein proximales Ende 56 eines Schaftrohrs 50 bis zu einer Montageposition (vgl. Figuren 10, 11) relativ zu einem ersten Bauteil 60, 70 eines als Teil der zu fertigenden Komponente zu fertigenden Schafts 15, 50, 60, 70, 80 in das erste Bauteil 60, 70 des Schafts 15, 50, 60, 70, 80 eingeführt. Die Montageposition des proximalen Endes 56 des Schaftrohrs 50 (vgl. Figuren 10, 11) befindet sich proximal der für die fertiggestellte Komponente vorgesehenen Position (vgl. Figur 7) des proximalen Endes 56 des Schaftrohrs 50 relativ zu dem ersten Bauteil 60, 70 des Schafts 15, 50, 60, 70, 80. Die Montageposition des proximalen Endes 56 des Schaftrohrs 50 kann sich proximal (vgl. Figuren 10, 11) oder distal des proximalen Endes des ersten Bauteils 60, 70 des Schafts 15, 50, 60, 70, 80 befinden.

Bei einem vierten Schritt 104 wird ein proximales Ende 23 eines ersten Bauteils 20 einer Übertragungseinrichtung 16, 20, 30, 40 von distal her bis zu einer Montageposition (vgl. Figuren 10, 11) durch das erste Bauteil 60, 70 des Schafts 15, 50, 60, 70, 80 hindurchgeführt. Die Montageposition des proximalen Endes 23 des ersten Bauteils 20 relativ zu dem ersten Bauteil 60, 70 des Schafts 15, 50, 60, 70, 80 liegt proximal seiner für die fertiggestellte Komponente 14 vorgesehenen Position (vgl. Figur 7). Die Montageposition des proximalen Endes 23 des ersten Bauteils 20 der Übertragungseinrichtung 160, 20, 30, 40 liegt insbesondere proximal des proximalen Endes des ersten Bauteils 60, 70 des Schafts 15, 50, 60, 70, 80.

Der erste Schritt 101, der zweite Schritt 102, der dritte Schritt 103 und der vierte Schritt 104 werden insbesondere in der angegebenen Reihenfolge ausgeführt. Alternativ können diese Schritte in einer anderen Reihenfolge ausgeführt werden, wobei der erste Schritt 101 und der zweite Schritt 102 insbesondere vor dem dritten Schritt 103 und / oder vor dem vierten Schritt 104 ausgeführt werden. Wenn der erste Schritt 101 und der zweite Schritt 102 vor dem dritten Schritt 103 und dem vierten Schritt 104 ausgeführt werden, können der dritte Schritt 103 und der vierte Schritt 104 gleichzeitig ausgeführt werden. Der erste Schritt 101, der zweite Schritt 102 und der dritte Schritt 103 sind jeweils optional und können entfallen.

Bei einem fünften Schritt 105 wird das proximale Ende 23 des ersten Bauteils 20 der Übertragungseinrichtung 16, 20, 30, 40 mit einem zweiten Bauteil 30, 40 der Übertragungseinrichtung 16, 20, 30, 40 mechanisch verbunden. Die bei dem fünften Schritt 105 hergestellte mechanische Verbindung ist insbesondere eine dauerhafte bzw. nicht zerstörungsfrei lösbare Verbindung. Das zweite Bauteil 30, 40 der Übertragungseinrichtung 16, 20, 30, 40 und das erste Bauteil 50, 60, 70 des Schafts 15, 50, 60, 70, 80 sind insbesondere so ausgebildet, dass eine Bewegung des zweiten Bauteils 30, 40 der Übertragungseinrichtung 16, 20, 30, 40 relativ zu dem ersten Bauteil 50, 60, 70 des Schafts 15, 50, 60, 70, 80 durch mechanischen Kontakt korrespondierender Anschlagflächen 37, 78 nach distal begrenzt ist.

Bei einem sechsten Schritt 106 wird das proximale Ende 23 des ersten Bauteils 20 der Übertragungseinrichtung 16, 20, 30, 40 ausgehend von der Montageposition des proximalen Endes 23 des ersten Bauteils 20 nach distal bis zu einer für die fertiggestellte Komponente vorgesehenen Position relativ zu dem ersten Bauteil 60, 70 des Schafts 15, 50, 60, 70, 80 bewegt.

Bei einem siebten Schritt 107 wird das proximale Ende 56 des Schaftrohrs 50 relativ zu dem ersten Bauteil des Schafts 15, 50, 60, 70, 80 nach distal bis zu der für die Verwendung der Komponente 14 vorgesehenen Position bewegt.

Der sechste Schritt 106 und der siebte Schritt 107 werden insbesondere gleichzeitig oder in beliebiger Reihenfolge, jedoch beide nach dem vierten Schritt 104 und dem fünften Schritt 105 ausgeführt.

Bei einem achten Schritt 108 wird das proximale Ende 56 des Schaftrohrs 50 mit dem ersten Bauteil 60, 70 des Schafts 15, 50, 60, 70, 80 verbunden. Der achte Schritt 108 wird insbesondere nach dem siebten Schritt 107, also bei der für die fertiggestellte Komponente 14 vorgesehenen Position des proximalen Endes 56 des Schaftrohrs 50 relativ zu dem ersten Bauteil 60, 70 des Schafts 15, 50, 60, 70, 80 ausgeführt.

### Bezugszeichen

- 10: medizinisches Instrument
- 13: Werkzeug an dem distalen Ende des medizinischen Instruments 10
- 14: Komponente des medizinischen Werkzeugs 10
- 15: Schaft des medizinischen Instruments 10 als Teil der Komponente 14
- 16: Übertragungseinrichtung des medizinischen Instruments 10 als Teil der Komponente 14
- 18: Handhabungseinrichtung an dem proximalen Ende des medizinischen Instruments 10
- 19: manuell bewegbarer Teil der Handhabungseinrichtung 18
- 20: Draht, einen langen dünnen Abschnitt der Übertragungseinrichtung 16 bildend, zur Anordnung in dem Schaftrohr 50 vorgesehen
- 23: proximales Ende des Drahts 20
- 30: erstes Stabbauteil der Übertragungseinrichtung 16
- 32: erste Längsbohrung an dem distalen Ende des ersten Stabbauteils 30 zur Aufnahme des proximalen Endes 23 des Drahts 20
- 33: Querbohrung zur Herstellung einer Schweißverbindung des proximalen Endes 23 des Drahts 20 mit dem ersten Stabbauteil 30
- 34: zweite Längsbohrung an dem proximalen Ende des ersten Stabbauteils 30 zur Aufnahme des Zapfens 43 an dem distalen Endes des zweiten Stabbauteils 40
- 35: Kragen oder Nase an dem ersten Stabbauteil 30
- 36: nach proximal orientierte Anschlagfläche der Übertragungseinrichtung 16, insb. Fläche an Kragen oder Nase 35 oder Stift 38
- 37: nach distal orientierte Anschlagfläche der Übertragungseinrichtung 16, insb. Fläche an Kragen oder Nase 3 5 oder Stift 38
- 38: Stift
- 39: Querbohrung in dem ersten Stabbauteil 30, zur Aufnahme des Stifts 38
- 40: zweites Stabbauteil der Übertragungseinrichtung 16
- 43: Zapfen an dem distalen Ende des zweiten Stabbauteils 40 zur mechanischen Verbindung mit dem ersten Stabbauteil 30
- 49: Kopplungskugel an dem proximalen Ende des zweiten Stabbauteils 40 zur Kopplung mit dem manuell bewegbaren Teil 19 der Handhabungseinrichtung 18
- 50: Schaftrohr des Schafts 15
- 52: Kanal in dem Schaftrohr 50 zur Aufnahme des Drahts 20
- 56: proximales Ende des Schaftrohrs 50
- 59: Spülöffnung in dem Schaftrohr 50
- 60: erstes Hülsenbauteil des Schafts 15, das proximale Ende 56 des Schaftrohrs 50 mit dem zweiten Hülsenbauteil 70 verbindend
- 65: Kanal in dem ersten Hülsenbauteil 60, zur Aufnahme des proximalen Endes 56 des Schaftrohrs 50
- 66: nach radial innen ragender Kragen an dem proximalen Ende des ersten Hülsenbauteils 60
- 67: Außengewinde an dem ersten Hülsenbauteil 60 zur mechanischen Verbindung mit dem zweiten Hülsenbauteil 70
- 70: zweites Hülsenbauteil des Schafts 15
- 73: Kanal in dem zweiten Hülsenbauteil 70, zur Aufnahme der Übertragungseinrichtung 16
- 74: O-Ring in dem zweiten Hülsenbauteil 70
- 76: Innengewinde an dem distalen Ende des zweiten Hülsenbauteil 70 zur mechanischen Verbindung mit dem Außengewinde 67 an dem ersten Hülsenbauteil 60
- 78: nach proximal orientierte Anschlagfläche des Schafts 15, insb. proximale Randfläche des zweiten Hülsenbauteils 70
- 80: drittes Hülsenbauteil des Schafts 15
- 83: Kanal in dem dritten Hülsenbauteil 80, zur Aufnahme der Übertragungseinrichtung 16
- 85: Ausnehmung in dem Schaft 15, zur Aufnahme des Kragens oder der Nase 35 oder eines Endes des Stifts 38 an dem ersten Stabbauteil 30
- 86: nach distal orientierte Anschlagfläche an dem Schaft 15, insb. nach distal orientierter Bereich der inneren Oberfläche der Ausnehmung 85
- 90: Anschlussbauteil des Schafts 15
- 95: Spülanschluss des Anschlussbauteils 90
- 101: erster Schritt (Koppeln eines distalen Endes einer Übertragungseinrichtung mit einem Werkzeug)
- 102: zweiter Schritt (Verbinden des Werkzeugs mit einem distalen Ende eines Schaftrohrs)
- 103: dritter Schritt (Einführen eines proximalen Endes eines Schaftrohrs in ein erstes Bauteil eines Schafts)
- 104: vierter Schritt (Hindurchführen eines proximalen Endes eines ersten Bauteils einer Übertragungseinrichtung)
- 105: fünfter Schritt (Verbinden des proximalen Endes des ersten Bauteils mit einem zweiten Bauteil der Übertragungseinrichtung)
- 106: sechster Schritt (Bewegen des proximalen Endes des ersten Bauteils der Übertragungseinrichtung nach distal bis zu der vorgesehenen Position)
- 107: siebter Schritt (Bewegen des proximalen Endes des Schaftrohrs nach distal bis zu der vorgesehenen Position)
- 108: achter Schritt (Verbinden des proximalen Endes des Schaftrohrs mit dem ersten Bauteil des Schafts)

## Patentansprüche

1. **Komponente** (14) zur mechanischen Verbindung mit einer Handhabungseinrichtung (18) zur Bildung eines medizinischen Instruments (10), mit:
einem **Schaft** (15);
einer **Übertragungseinrichtung** (16), die in dem Schaft (15) bewegbar ist, zum Übertragen einer Kraft von einer mit dem proximalen Ende der Komponente (14) gekoppelten Handhabungseinrichtung (18) zu einem Werkzeug (13) an dem distalen Ende der Komponente (14);
einer **Anschlagfläche** (86) an dem Schaft (15),
einer **Anschlagfläche** (36) an der Übertragungseinrichtung (16),
wobei die Anschlagfläche (86) des Schafts (15) und die Anschlagfläche (36) der Übertragungseinrichtung (16) so angeordnet sind, dass ein mechanischer Kontakt zwischen der Anschlagfläche (36) der Übertragungseinrichtung (16) und der Anschlagfläche (86) des Schafts (15) eine Bewegung der Übertragungseinrichtung (16) relativ zu dem Schaft (15) **nach proximal begrenzt,**
**dadurch gekennzeichnet dass**,
die Übertragungseinrichtung (16) in einem Abschnitt (20) distal der Anschlagfläche (36) der Übertragungseinrichtung (16) ihre **minimale Querschnittsfläche** aufweist,
alle Querschnittsflächen der Übertragungseinrichtung (16) **proximal** der Anschlagfläche (36) der Übertragungseinrichtung (16) **größer** ist als die minimale Querschnittsfläche der Übertragungseinrichtung (16).

2. Komponente (14) nach dem vorangehenden Anspruch, bei der
die Anschlagfläche (86) des Schafts (15) und die Anschlagfläche (36) der Übertragungseinrichtung (16) so angeordnet sind, dass ein mechanischer Kontakt zwischen der Anschlagfläche (36) der Übertragungseinrichtung (16) und der Anschlagfläche (86) des Schafts (15) bei der vorgesehenen Verwendung eine **elastische Verformung** der Übertragungseinrichtung (16) durch eine von der Übertragungseinrichtung (16) übertragene Kraft **auf einen vorbestimmten Maximalwert begrenzt,**
der vorbestimmte Maximalwert der elastischen Verformung und **elastische Eigenschaften** der Übertragungseinrichtung (16) so gewählt sind, dass bei der Kraft, die bei dem vorbestimmten Maximalwert der elastischen Verformung der Übertragungseinrichtung (16) vorliegt, eine Zerstörung oder **Schädigung** des Schafts (15), der Übertragungseinrichtung (16) und eines mit dem distalen Ende des Schafts (15) verbundenen und mit dem distalen Ende der Übertragungseinrichtung (16) gekoppelten Werkzeugs (13) **ausgeschlossen** ist.

3. Komponente (14) nach dem vorangehenden Anspruch, bei der eine Zerstörung oder **Schädigung** des Schafts (15), der Übertragungseinrichtung (16) und eines mit dem distalen Ende des Schafts (15) gekoppelten Werkzeugs (13) auch dann **ausgeschlossen** ist, wenn das Werkzeug (13) in einem ganz geöffneten Zustand oder in einem ganz geschlossenen Zustand oder in einem beliebigen Zustand blockiert ist.

4. Komponente (14) nach einem der vorangehenden Ansprüche, bei der die Anschlagfläche (86) des Schafts (15) nahe dem **proximalen Ende** des Schafts (15) und die Anschlagfläche (36) der Übertragungseinrichtung (16) nahe dem proximalen Ende der Übertragungseinrichtung (16) angeordnet sind.

5. Komponente (14) nach einem der vorangehenden Ansprüche, bei der
die Übertragungseinrichtung (16) distal der Anschlagfläche (36) der Übertragungseinrichtung (16) bis zu einer ersten **Maximalkraft** elastisch verformbar ist,
die Übertragungseinrichtung (16) proximal der Anschlagfläche (36) der Übertragungseinrichtung (16) bis zu einer zweiten **Maximalkraft** elastisch verformbar ist,
die zweite Maximalkraft größer ist als die erste Maximalkraft.

6. Komponente (14) nach einem der vorangehenden Ansprüche, ferner mit:
einer **weiteren Anschlagfläche** (78) an dem Schaft (15),
einer **weiteren Anschlagfläche** (37) an der Übertragungseinrichtung (16),
wobei die weitere Anschlagfläche (78) des Schafts (15) und die weitere Anschlagfläche (37) der Übertragungseinrichtung (16) so angeordnet sind, dass ein mechanischer Kontakt zwischen der weiteren Anschlagfläche (37) der Übertragungseinrichtung (16) und der weiteren Anschlagfläche (78) des Schafts (15) eine Bewegung der Übertragungseinrichtung (16) relativ zu dem Schaft (15) nach distal begrenzt.

7. Komponente (14) nach einem der vorangehenden Ansprüche, bei der
die Übertragungseinrichtung (16) einen nach außen ragenden Kragen (35) aufweist,
der Schaft (15) eine Nut (85), die einen Kanal (83), in dem die Übertragungseinrichtung (16) angeordnet ist, erweitert, aufweist,
der Kragen (35) der Übertragungseinrichtung (16) in der Nut (85) des Schafts (15) angeordnet ist,
die Anschlagfläche (86) des Schafts (15) Teil der inneren Oberfläche der Nut (85) ist,
die Anschlagfläche (36) der Übertragungseinrichtung (16) Teil der Oberfläche des Kragens (35) ist.

8. Komponente (14) nach einem der vorangehenden Ansprüche, bei der
die Übertragungseinrichtung (16) einen Vorsprung, eine Nase, einen Steg, einen Zapfen oder einen anderen konvexen Bereich (38) aufweist,
der Schaft (15) eine Nut oder eine andere nischenförmige Ausnehmung (85), die von einem Kanal (83), in dem die Übertragungseinrichtung (16) angeordnet ist, ausgeht, aufweist
der Vorsprung oder die Nase oder der Steg oder der Zapfen oder der andere konvexe Bereich (38) der Übertragungseinrichtung (16) in der Nut oder der anderen nischenförmigen Ausnehmung (85) des Schafts (15) angeordnet ist,
die Anschlagfläche (86) des Schafts (15) Teil der inneren Oberfläche der Nut oder der anderen nischenförmigen Ausnehmung (85) des Schafts (15) ist,
die Anschlagfläche (36) der Übertragungseinrichtung (16) Teil der Oberfläche des Vorsprungs oder der Nase oder des Stegs oder des Zapfens oder des anderen konvexen Bereichs (38) ist.

9. Komponente (14) nach einem der vorangehenden Ansprüche, ferner mit:
einem **Werkzeug** (13) an dem distalen Ende des Schafts (15),
wobei das Werkzeug (13) ein **bewegbares Bauteil,** das mit dem distalen Ende der Übertragungseinrichtung (16) gekoppelt ist, aufweist.

10. **Medizinisches Instrument** (10) mit:
einer **Komponente** (14) nach einem der vorangehenden Ansprüche;
einer **Handhabungseinrichtung** (18), die mit dem proximalen Ende des Schafts (15) gekoppelt oder koppelbar ist.

11. **Verfahren zum Herstellen** einer mit einer Handhabungseinrichtung (18) mechanisch verbindbaren Komponente (14) gemäß einem der Ansprüche 1 bis 9 für ein medizinisches Instrument (10) mit folgenden Schritten:
**Hindurchführen** (104) eines proximalen Endes (23) eines ersten Bauteils (20) einer Übertragungseinrichtung (16, 20, 30, 40) von distal durch ein erstes Bauteil (60, 70) eines Schafts (15, 50, 60, 70, 80) bis zu einer Montageposition, die proximal der für die fertiggestellte Komponente (14) vorgesehenen Position relativ zu dem ersten Bauteil (60, 70) des Schafts (15, 50, 60, 70, 80) liegt;
mechanisches **Verbinden** (105) des proximalen Endes (23) des ersten Bauteils (20) der Übertragungseinrichtung (16, 20, 30, 40) mit einem zweiten Bauteil (30, 40) der Übertragungseinrichtung (16, 20, 30, 40);
**Bewegen** (106) des proximalen Endes (23) des ersten Bauteils (20) der Übertragungseinrichtung (16, 20, 30, 40) nach distal bis zu einer für die fertiggestellte Komponente (14) vorgesehenen Position relativ zu dem ersten Bauteil (60, 70) des Schafts (15, 50, 60, 70, 80).

12. Verfahren nach dem vorangehenden Anspruch, ferner mit folgenden Schritten:
**Einführen** (103) eines proximalen Endes (56) eines Schaftrohrs (50) in das erste Bauteil (60, 70) des Schafts (15, 50, 60, 70, 80) **bis zu einer Montageposition, die proximal** der für die fertiggestellte Komponente (14) vorgesehenen Position relativ zu dem ersten Bauteil (60, 70) des Schafts (15, 50, 60, 70, 80) liegt, **vor dem Verbinden** (10) des proximalen Endes (23) des ersten Bauteils (20) der Übertragungseinrichtung (16, 20, 30, 40) mit dem zweiten Bauteil (30, 40) der Übertragungseinrichtung (16, 20, 30, 40);
Bewegen (107) des proximalen Endes (56) des Schaftrohrs (50) relativ zu dem ersten Bauteil (60, 70) des Schafts (15, 50, 60, 70, 80) nach distal bis zu der für die Verwendung der Komponente (14) vorgesehenen Position (60, 70) des Schaftrohrs (50) relativ zu dem ersten Bauteil (60, 70) des Schafts (15, 50, 60, 70, 80) **nach dem Verbinden** (10) des proximalen Endes (23) des ersten Bauteils (20) der Übertragungseinrichtung (16, 20, 30, 40) mit einem zweiten Bauteil (30, 40) der Übertragungseinrichtung (16, 20, 30, 40);
mechanisches **Verbinden** (108) des proximalen Endes (56) des Schaftrohrs (50) mit dem ersten Bauteil (60, 70) des Schafts (15, 50, 60, 70, 80) an der für die fertiggestellte Komponente (14) vorgesehenen Position relativ zu dem ersten Bauteil (60, 70) des Schafts (15, 50, 60, 70, 80).

13. Verfahren nach einem der Ansprüche 11 und 12, ferner mit folgenden Schritten:
**Koppeln** (101) eines distalen Endes der Übertragungseinrichtung (16, 20, 30, 40) mit einem bewegbaren Teil eines Werkzeugs (13);
mechanisch **Verbinden** (102) des Werkzeugs (13) mit einem distalen Ende des Schafts (15, 50, 60, 70, 80).

14. Verfahren nach dem vorangehenden Anspruch, bei dem zumindest entweder der Schritt des Koppelns (101) des distalen Endes der Übertragungseinrichtung (16, 20, 30, 40) mit einem bewegbaren Teil des Werkzeugs (13) oder der Schritt des mechanischen Verbindens (102) des Werkzeugs (13) mit dem distalen Ende des Schafts (15, 50, 60, 70, 80) zumindest entweder **vor** dem Schritt des **Einführens** (103) des proximalen Endes (56) des Schaftrohrs (50) in das erste Bauteil (60, 70) des Schafts (15, 50, 60, 70, 80) oder vor dem Schritt des **Hindurchführens** (104) des proximalen Endes (23) des ersten Bauteils (20) der Übertragungseinrichtung (16, 20, 30, 40) von distal durch das erste Bauteil (60, 70) des Schafts (15, 50, 60, 70, 80) ausgeführt werden.

## Claims

1. A component (14) for mechanical connection to a handling device (18) to form a medical instrument (10), comprising:
a shaft (15);
a transmission device (16) which can be moved in the shaft (15) for transmitting a force from a handling device (18) coupled to the proximal end of the component (14) to a tool (13) at the distal end of the component (14);
an abutment surface (86) on the shaft (15);
an abutment surface (36) on the transmission device (16),
wherein the abutment surface (86) of the shaft (15) and the abutment surface (36) of the transmission device (16) are arranged such that a mechanical contact between the abutment surface (36) of the transmission device (16) and the abutment surface (86) of the shaft (15) proximally limits a movement of the transmission device (16) relative to the shaft (15),
**characterised in that** the transmission device (16) has its minimum cross-sectional area in a section (20) distal to the abutment surface (36) of the transmission device (16),
all cross-sectional areas of the transmission device (16) proximal to the abutment surface (36) of the transmission device (16) are greater than the minimum cross-sectional area of the transmission device (16).

2. The component (14) according to the preceding claim, in which
the abutment surface (86) of the shaft (15) and the abutment surface (36) of the transmission device (16) are arranged such that a mechanical contact between the abutment surface (36) of the transmission device (16) and the abutment surface (86) of the shaft (15) during the intended use limits an elastic deformation of the transmission device (16) through a force transmitted by the transmission device (16) to a predetermined maximum value,
the predetermined maximum value of the elastic deformation and elastic properties of the transmission device (16) are selected such that, in the case of the force which is present at the predetermined maximum value of the elastic deformation of the transmission device (16), destruction or damage of the shaft (15), the transmission device (16) and a tool (13) connected to the distal end of the shaft (15) and coupled to the distal end of the transmission device (16) is excluded.

3. The component (14) according to the preceding claim, in which destruction or damage of the shaft (15), the transmission device (16) and a tool (13) coupled to the distal end of the shaft (15) is even excluded when the tool (13) is blocked in a fully open state or in a fully closed state or in any state.

4. The component (14) according to one of the preceding claims, in which the abutment surface (86) of the shaft (15) is arranged near the proximal end of the shaft (15) and the abutment surface (36) of the transmission device (16) is arranged near the proximal end of the transmission device (16).

5. The component (14) according to one of the preceding claims, in which
the transmission device (16) is elastically deformable distally to the abutment surface (36) of the transmission device (16) up to a first maximum force,
the transmission device (16) is elastically deformable proximally to the abutment surface (36) of the transmission device (16) up to a second maximum force,
the second maximum force is greater than the first maximum force.

6. The component (14) according to one of the preceding claims, further comprising:
a further abutment surface (78) on the shaft (15);
a further abutment surface (37) on the transmission device (16),
wherein the further abutment surface (78) of the shaft (15) and the further abutment surface (37) of the transmission device (16) are arranged such that a mechanical contact between the further abutment surface (37) of the transmission device (16) and the further abutment surface (78) of the shaft (15) distally limits a movement of the transmission device (16) relative to the shaft (15).

7. The component (14) according to one of the preceding claims, in which
the transmission device (16) has an outwardly projecting collar (35),
the shaft (15) has a groove (85) which widens a channel (83) in which the transmission device (16) is arranged,
the collar (35) of the transmission device (16) is arranged in the groove (85) of the shaft (15),
the abutment surface (86) of the shaft (15) is part of the inner surface of the groove (85),
the abutment surface (36) of the transmission device (16) is part of the surface of the collar (35).

8. The component (14) according to one of the preceding claims, in which
the transmission device (16) has a projection, a nose, a web, a pin or another convex region (38),
the shaft (15) has a groove or other niche-shaped recess (85) which extends from a channel (83) in which the transmission device (16) is arranged
the projection or the nose or the web or the pin or the other convex region (38) of the transmission device (16) is arranged in the groove or the other niche-shaped recess (85) of the shaft (15),
the abutment surface (86) of the shaft (15) is part of the inner surface of the groove or other niche-shaped recess (85) of the shaft (15),
the abutment surface (36) of the transmission device (16) is part of the surface of the projection or the nose or the web or the pin or the other convex region (38).

9. The component (14) according to one of the preceding claims, further comprising:
a tool (13) at the distal end of the shaft (15),
wherein the tool (13) has a movable component which is coupled to the distal end of the transmission device (16).

10. A medical instrument (10) having:
a component (14) according to one of the preceding claims;
a handling device (18) which is or can be coupled to the proximal end of the shaft (15).

11. A method for manufacturing a component (14) mechanically connectable to a handling device (18) according to one of claims 1 to 9 for a medical instrument (10), having the following steps:
guiding (104) a proximal end (23) of a first component (20) of a transmission device (16, 20, 30, 40) from distal through a first component (60, 70) of a shaft (15, 50, 60, 70, 80) to an assembly position which is proximal to the position provided for the finished component (14) relative to the first component (60, 70) of the shaft (15, 50, 60, 70, 80);
mechanically connecting (105) the proximal end (23) of the first component (20) of the transmission device (16, 20, 30, 40) to a second component (30, 40) of the transmission device (16, 20, 30, 40);
moving (106) the proximal end (23) of the first component (20) of the transmission device (16, 20, 30, 40) distally to a position intended for the finished component (14) relative to the first component (60, 70) of the shaft (15, 50, 60, 70, 80).

12. The method according to the preceding claim, further comprising the following steps:
introducing (103) a proximal end (56) of a shaft tube (50) into the first component (60, 70) of the shaft (15, 50, 60, 70, 80) to an assembly position that is proximal to the position intended for the finished component (14) relative to the first component (60, 70) of the shaft (15, 50, 60, 70, 80), before connecting (10) the proximal end (23) of the first component (20) of the transmission device (16, 20, 30, 40) to the second component (30, 40) of the transmission device (16, 20, 30, 40);
moving (107) the proximal end (56) of the shaft tube (50) relative to the first component (60, 70) of the shaft (15, 50, 60, 70, 80) distally to the position (60, 70) of the shaft tube (50) intended for use of the component (14) relative to the first component (60, 70) of the shaft (15, 50, 60, 70, 80) after connecting (10) the proximal end (23) of the first component (20) of the transmission device (16, 20, 30, 40) to a second component (30, 40) of the transmission device (16, 20, 30, 40);
mechanically connecting (108) the proximal end (56) of the shaft tube (50) to the first component (60, 70) of the shaft (15, 50, 60, 70, 80) at the position intended for the finished component (14) relative to the first component (60, 70) of the shaft (15, 50, 60, 70, 80).

13. The method according to one of claims 11 and 12, further comprising the following steps:
coupling (101) a distal end of the transmission device (16, 20, 30, 40) to a movable part of a tool (13);
mechanically connecting (102) the tool (13) to a distal end of the shaft (15, 50, 60, 70, 80).

14. The method according to the preceding claim, in which at least either the step of coupling (101) the distal end of the transmission device (16, 20, 30, 40) to a movable part of the tool (13) or the step of mechanically connecting (102) the tool (13) to the distal end of the shaft (15, 50, 60, 70, 80) at least either before the step of introducing (103) the proximal end (56) of the shaft tube (50) into the first component (60, 70) of the shaft (15, 50, 60, 70, 80) or before the step of guiding (104) the proximal end (23) of the first component (20) of the transmission device (16, 20, 30, 40) from distal through the first component (60, 70) of the shaft (15, 50, 60, 70, 80).

## Revendications

1. **Composant** (14) pour la liaison mécanique à une installation de manipulation (18) pour la formation d'un instrument médical (10), comportant :
un **arbre** (15) ;
une **installation de transmission** (16) mobile à l'intérieur de l'arbre (15) pour la transmission d'une force depuis une installation de manipulation (18) accouplée à l'extrémité proximale du composant (14) à un outil (13) à l'extrémité distale du composant (14) ;
une **surface de butée** (86) sur l'arbre (15) ;
une **surface de butée** (36) sur l'installation de transmission (16),
dans lequel la surface de butée (86) de l'arbre (15) et la surface de butée (36) de l'installation de transmission (16) sont agencées de telle sorte qu'un contact mécanique entre la surface de butée (36) de l'installation de transmission (16) et la surface de butée (86) de l'arbre (15) limite un mouvement de l'installation de transmission (16) par rapport à l'arbre (15) **vers le côté proximal,**
**caractérisé en ce que** l'installation de transmission (16) présente, dans une section (20) distale par rapport à la surface de butée (36) de l'installation de transmission (16) sa **surface en section transversale minimale,**
toutes les surfaces en section transversale de l'installation de transmission (16) **proximales** par rapport à la surface de butée (36) de l'installation de transmission (16) sont **plus grandes** que la surface en section transversale minimale de l'installation de transmission (16).

2. Composant (14) selon la revendication précédente, dans lequel
la surface de butée (86) de l'arbre (15) et la surface de butée (36) de l'installation de transmission (16) sont agencées de telle sorte qu'un contact mécanique entre la surface de butée (36) de l'installation de transmission (16) et la surface de butée (86) de l'arbre (15) limite, lors de l'utilisation prévue, une **déformation élastique** de l'installation de transmission (16) par une force transmise par l'installation de transmission (16) **à une valeur maximale prédéterminée,**
la valeur maximale prédéterminée de déformation élastique et les **propriétés élastiques** de l'installation de transmission (16) sont choisis de manière à ce que, pour la force présente à la valeur maximale prédéterminée de la déformation élastique de l'installation de transmission (16), une destruction ou un **endommagement** de l'arbre (15), de l'installation de transmission (16) et d'un outil (13) relié à l'extrémité distale de l'arbre (15) et accouplé à l'extrémité distale de l'installation de transmission (16) sont **exclus.**

3. Composant (14) selon la revendication précédente, dans lequel la destruction ou l'**endommagement** de l'arbre (15), de l'installation de transmission (16) et d'un outil (13) accouplé à l'extrémité distale de l'arbre (15) sont également **exclus** lorsque l'outil (13) est bloqué dans un état complètement ouvert ou dans un état complètement fermé ou dans un état quelconque.

4. Composant (14) selon l'une des revendications précédentes, dans lequel la surface de butée (86) de l'arbre (15) est agencée à proximité de **l'extrémité proximale** de l'arbre (15) et la surface de butée (36) de l'installation de transmission (16) est agencée à proximité de l'extrémité proximale de l'installation de transmission (16).

5. Composant (14) selon l'une des revendications précédentes, dans lequel
l'installation de transmission (16) est élastiquement déformable de manière distale par rapport à la surface de butée (36) de l'installation de transmission (16) jusqu'à une première **force maximale,**
l'installation de transmission (16) est élastiquement déformable de manière proximale par rapport à la surface de butée (36) de l'installation de transmission (16) à une seconde **force maximale,**
la seconde force maximale est supérieure à la première force maximale.

6. Composant (14) selon l'une des revendications précédentes, comportant en outre :
une **surface de butée supplémentaire** (78) sur l'arbre (15) ;
une **surface de butée supplémentaire** (37) sur l'installation de transmission (16),
dans lequel la surface de butée supplémentaire (78) de l'arbre (15) et la surface de butée supplémentaire (37) de l'installation de transmission (16) sont agencées de telle sorte qu'un contact mécanique entre la surface de butée supplémentaire (37) de l'installation de transmission (16) et la surface de butée supplémentaire (78) de l'arbre (15) limite un mouvement de l'installation de transmission (16) par rapport à l'arbre (15) vers le côté distal.

7. Composant (14) selon l'une des revendications précédentes, dans lequel
l'installation de transmission (16) présente une collerette (35) faisant saillie vers l'extérieur,
l'arbre (15) présente une rainure (85) qui élargit un canal (83) dans lequel est agencée l'installation de transmission (16),
la collerette (35) de l'installation de transmission (16) est agencée dans la rainure (85) de l'arbre (15),
la surface de butée (86) de l'arbre (15) fait partie de la surface intérieure de la rainure (85),
la surface de butée (36) de l'installation de transmission (16) fait partie de la surface de la collerette (35).

8. Composant (14) selon l'une des revendications précédentes, dans lequel
l'installation de transmission (16) présente une saillie, un nez, un nervure, un tenon ou une autre zone convexe (38),
l'arbre (15) présente une rainure ou un autre évidement (85) en forme de niche qui s'étend depuis un canal (83) dans lequel est agencée l'installation de transmission (16) la saillie ou le nez ou la nervure ou le tenon ou l'autre zone convexe (38) de l'installation de transmission (16) est agencé dans la rainure ou l'autre évidement (85) en forme de niche de l'arbre (15),
la surface de butée (86) de l'arbre (15) fait partie de la surface intérieure de la rainure ou de l'autre évidement (85) en forme de niche de l'arbre (15),
la surface de butée (36) de l'installation de transmission (16) fait partie de la surface de la saillie ou du nez ou de la nervure ou du tenon ou de l'autre zone convexe (38).

9. Composant (14) selon l'une des revendications précédentes, comportant en outre :
un **outil** (13) à l'extrémité distale de l'arbre (15),
dans lequel l'outil (13) présente un **élément de construction mobile** qui est accouplé à l'extrémité distale de l'installation de transmission (16).

10. **Instrument médical** (10) comportant :
un **composant** (14) selon l'une des revendications précédentes ;
une **installation de manipulation** (18), qui est accouplée ou peut être couplée à l'extrémité proximale de l'arbre (15).

11. **Procédé de fabrication** d'un composant (14) pouvant être relié mécaniquement à une installation de manipulation (18) selon l'une des revendications 1 à 9 pour un instrument médical (10) comportant les étapes suivantes :
**passage** (104) d'une extrémité proximale (23) d'un premier élément de construction (20) d'une installation de transmission (16, 20, 30, 40) depuis le côté distal à travers un premier élément de construction (60, 70) d'un arbre (15, 50, 60 , 70, 80) vers une position d'assemblage qui est proximale à la position prévue pour le composant (14) fini par rapport au premier élément de construction (60, 70) de l'arbre (15, 50, 60, 70, 80) ;
**liaison** mécanique (105) de l'extrémité proximale (23) du premier élément de construction (20) de l'installation de transmission (16, 20, 30, 40) avec un second élément de construction (30, 40) de l'installation de transmission (16, 20, 30, 40) ;
**déplacement** (106) de l'extrémité proximale (23) du premier élément de construction (20) de l'installation de transmission (16, 20, 30, 40) vers le côté distal jusqu'à une position prévue pour le composant (14) fini par rapport au premier élément de construction (60, 70) de l'arbre (15, 50, 60, 70, 80).

12. Procédé selon la revendication précédente, comportant en outre les étapes suivantes :
**introduction** (103) d'une extrémité proximale (56) d'un tube d'arbre (50) dans le premier élément de construction (60, 70) de l'arbre (15, 50, 60, 70, 80) **jusqu'à une position de montage qui est proximale** à la position prévue pour le composant (14) fini par rapport au premier élément de construction (60, 70) de l'arbre (15, 50, 60, 70, 80), **avant la liaison** (10) de l'extrémité proximale (23) du premier élément de construction (20) de l'installation de transmission (16, 20, 30, 40) avec le second élément de construction (30, 40) de l'installation de transmission (16, 20, 30, 40) ;
déplacement (107) de l'extrémité proximale (56) du tube d'arbre (50) par rapport au premier élément de construction (60, 70) de l'arbre (15, 50, 60, 70, 80) vers le côté distal jusqu'à la position (60, 70) prévue pour l'utilisation du composant (14) du tube d'arbre (50) par rapport au premier élément de construction (60, 70) de l'arbre (15, 50, 60, 70, 80) **après la liaison** (10) de l'extrémité proximale (23) du premier élément de construction (20) de l'installation de transmission (16, 20, 30, 40) avec un second élément de construction (30, 40) de l'installation de transmission (16, 20, 30, 40) ;
**liaison** mécanique (108) de l'extrémité proximale (56) du tube d'arbre (50) avec le premier élément de construction (60, 70) de l'arbre (15, 50, 60, 70, 80) à la position prévue pour le composant (14) fini par rapport au premier élément de construction (60, 70) de l'arbre (15, 50, 60, 70, 80).

13. Procédé selon l'une des revendications 11 et 12, comportant en outre les étapes suivantes :
**accouplement** (101) d'une extrémité distale de l'installation de transmission (16, 20, 30, 40) avec une partie mobile d'un outil (13) ;
**liaison** mécanique (102) de l'outil (13) avec une extrémité distale de l'arbre (15, 50, 60, 70, 80).

14. Procédé selon la revendication précédente, dans lequel au moins soit l'étape d'accouplement (101) de l'extrémité distale de l'installation de
transmission (16, 20, 30, 40) avec une partie mobile de l'outil (13) soit l'étape de liaison mécanique (102) de l'outil (13) à l'extrémité distale de l'arbre (15, 50, 60, 70, 80) sont effectuées au moins soit **avant** l'étape **d'introduction** (103) de l'extrémité proximale (56) du tube d'arbre (50) dans le premier élément de construction (60, 70) de
l'arbre (15, 50, 60, 70, 80) soit avant l'étape de **passage** (104) de l'extrémité proximale (23) du premier élément de construction (20) de l'installation de
transmission (16, 20, 30, 40) depuis le côté distal à travers le premier élément de construction (60, 70) de l'arbre (15, 50, 60, 70, 80).
